# EUROPÄISCHE PATENTSCHRIFT

(11) **EP 1 239 828 B1**
(45) Veröffentlichungstag und Bekanntmachung des Hinweises auf die Patenterteilung: **05.04.2006**
(21) Anmeldenummer: 00983312.0
(22) Anmeldetag: 13.12.2000
(51) Int. Cl.: C11D 3/00, C11D 1/62, A61K 8/04, A61K 8/41, A61Q 5/12

(54) **VERWENDUNG VON KATIONISCHEN VERBINDUNGEN**
USE OF CATIONIC COMPOUNDS
UTILISATION DE COMPOSES CATIONIQUES

(30) Priorität: 22.12.1999 DE 19961939
(43) Veröffentlichungstag der Anmeldung: 18.09.2002
(73) Patentinhaber: Cognis IP Management GmbH, 40589 Düsseldorf (DE)
(72) Erfinder: HENSEN, Hermann, 42781 Haan (DE); EGGERS, Anke, 40215 Düsseldorf (DE); KAHRE, Jörg, 42799 Leichlingen (DE); BÖTTCHER, Axel, 41569 Rommerskirchen (DE)
(86) Internationale Anmeldenummer: PCT/EP2000/012655
(87) Internationale Veröffentlichungsnummer: WO 2001/045666

(56) Entgegenhaltungen:
- WO-A-00/47177
- WO-A-96/20698
- ZULLI ET AL: "Preparation and properties of small nanoparticles for skin and hair care" SOFW-JOURNAL SEIFEN, OELE, FETTE, WACHSE,DE,VERLAG FUR CHEMISCHE INDUSTRIE, H. ZIOLKOWSKY K.G. AUGSBURG, Bd. 123, Nr. 13, 1997, Seiten 880,883-885, XP002079030 ISSN: 0942-7694

## Beschreibung

Die Erfindung befindet sich auf dem Gebiet der Nanopartikel und betrifft die Verwendung von nanoskaligen kationischen Verbindungen in der Kosmetik.

### Stand der Technik

Kationische Verbindungen, wie beispielsweise Esterquats, quartäre Ammoniumverbindungen und dergleichen gewinnen wegen ihrer ausgezeichneten ökotoxikologischen Eigenschaften sowohl für den Bereich der WäscheweichspütmittPl als auch für kosmetische Anwendungen zunehmend an Bedeutung. In kosmetischen Zubereitungen werden diese Verbindungen zur Erzielung eines angenehmen weichen Haut- und Haargefühls eingesetzt. Sie können dabei sowohl in Emulsionen und Lotionen zur Hautpflege wie auch in tensidischen Mitteln wie beispielsweise Shampoos, Duschbädem, Spülungen, Conditionem und dergleichen für die Haarpflege enthalten sein.
Die Wirkung dieser kationischen Verbindungen hängt stets mit der Geschwindigkeit zusammen, mit der die Verbindungen eingebaut bzw. resorbiert werden. Hier besteht für die verfügbaren Stoffe des Stands der Technik noch ein erhebliches Verbesserungspotential.

Die Aufgabe der Erfindung hat somit darin bestanden, die Aufnahme von kationischen Verbindungen bei ihrer Applikation durch Bereitstellung neuer Anbietungsformen zu beschleunigen. Weiterhin sollen sie nach der Anwendung eine lang anhaltende Wirkung zeigen, eine gute dermatologische Verträglichkeit aufweisen und sich durch eine hervorragende Stabilität bei Temperaturlagerung auszeichnen.

### Beschreibung der Erfindung

Gegenstand der Erfindung ist die Verwendung von nanoskaligen Esterquats, Tetraalkylammonium verbindungen und/oder kationischen Polymeren im Bereich von 10 bis 300 nm zur Herstellung von kosmetischen und/oder pharmazeutischen Zubereitungen.

Überraschenderweise wurde gefunden, daß sich die Resorption von kationischen Verbindungen, wie Esterquats, Tetraalkylammoniumverbindungen und/oder kationischen Polymeren durch die Keratinfibrillen des Haares signifikant steigem läßt und damit der Weichgriffs der Haare verbessert wird, wenn diese in Form von Nanoteilchen, d.h. Partikeln mit einem Durchmesser im Bereich von 10 bis 300 und vorzugsweise 50 bis 150 nm vorliegen. Weiterhin erzielen diese Verbindungen ein angenehm weiches Hautgefühl und zeigen ebenfalls positive Effekte bei Verwendung in Wäscheweichspülmitteln. Weiterhin wird sowohl die Stabilität von Lotionen und Cremes als auch deren Konsistenz durch den Zusatz von nanoskaligen kationischen Verbindungen signifikant verbessert.

### Kationische Verbindungen

Im Sinne der Erfindung kommen als kationische Verbindungen Esterquats, Tetraalkylammoniumverbindungen und/oder kationische Polymere in Frage.

### Esterquats

Unter der Bezeichnung "Esterquats" werden im allgemeinen quatemierte Fettsäuretriethanolaminestersalze verstanden. Es handelt sich dabei um bekannte Stoffe, die man nach den einschlägigen Methoden der präparativen organischen Chemie erhalten kann. In diesem Zusammenhang sei auf die Internationale Patentanmeldung **WO 91/01295** verwiesen, nach der man Triethanolamin in Gegenwart von unterphosphoriger Säure mit Fettsäuren partiell verestert, Luft durchleitet und anschließend mit Dimethylsulfat oder Ethylenoxid quatemiert. Aus der Deutschen Patentschrift **DE 4308794 C1** ist überdies ein Verfahren zur Herstellung fester Esterquats bekannt, bei dem man die Quatemierung von Triethanolaminestem in Gegenwart von geeigneten Dispergatoren, vorzugsweise Fettalkoholen, durchführt. Übersichten zu diesem Thema sind beispielsweise von R.Puchta et al. in **Tens.Surf.Det., 30, 186** (1993)**,** M.Brock in **Tens.Surf.Det. 30, 394 (1993)**, R.Lagerman et al. in **J.Am. Oil.Chem.Soc., 71, 97 (1994)** sowie I.Shapiro in **Cosm.Toil. 109, 77 (1994)** erschienen.

Die quatemierten Fettsäuretriethanolaminestersalze folgen der Formel (I), 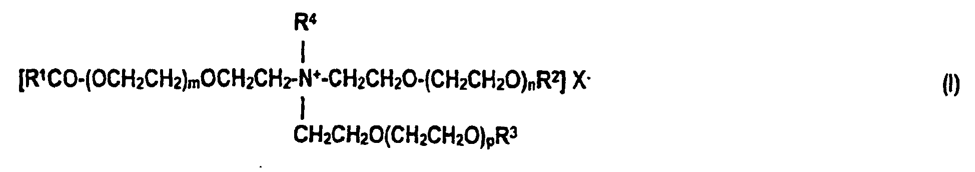 in der R¹CO für einen Acylrest mit 6 bis 22 Kohlenstoffatomen, R² und R³ unabhängig voneinander für Wasserstoff oder R¹CO, R⁴ für einen Alkylrest mit 1 bis 4 Kohlenstoffatomen oder eine (CH₂CH₂O)_{q}H-Gruppe, m, n und p in Summe für 0 oder Zahlen von 1 bis 12, q für Zahlen von 1 bis 12 und X für Halogenid, Alkylsulfat oder Alkylphosphat steht. Typische Beispiele für Esterquats, die im Sinne der Erfindung Verwendung finden können, sind Produkte auf Basis von Capronsäure, Caprylsäure, Caprinsäure, Laurinsäure, Myristinsäure, Palmitinsäure, Isostearinsäure, Stearinsäure, Ölsäure, Elaidinsäure, Arachinsäure, Behensäure und Erucasäure sowie deren technische Mischungen, wie sie beispielsweise bei der Druckspaltung natürlicher Fette und Öle anfallen. Vorzugsweise werden technische C_{12/18}-Kokosfettsäuren und insbesondere teilgehärtete C_{16/18}-Talg- bzw. Palmfettsäuren sowie elaidinsäurereiche C_{16/18}-Fettsäureschnitte eingesetzt. Zur Herstellung der quatemierten Ester können die Fettsäuren und das Triethanolamin im molaren Verhältnis von 1,1 : 1 bis 3 : 1 eingesetzt werden. Im Hinblick auf die anwendungstechnischen Eigenschaften der Esterquats hat sich ein Einsatzverhältnis von 1,2 : 1 bis 2,2 : 1, vorzugsweise 1,5 : 1 bis 1,9 : 1 als besonders vorteilhaft erwiesen. Die bevorzugten Esterquats stellen technische Mischungen von Mono-, Di- und Triestern mit einem durchschnittlichen Veresterungsgrad von 1,5 bis 1,9 dar und leiten sich von technischer C_{16/18}- Talg- bzw. Palmfettsäure (lodzahl 0 bis 40) ab. Aus anwendungstechnischer Sicht haben sich quatemierte Fettsäuretriethanolaminestersalze der Formel (1) als besonders vorteilhaft erwiesen, in der R¹CO für einen Acylrest mit 16 bis 18 Kohlenstoffatomen, R² für R¹CO, R³ für Wasserstoff, R⁴ für eine Methylgruppe, m, n und p für 0 und X für Methylsulfat steht. Entsprechende Produkte sind unter der Marke Dehyquart® AU (Cognis Deutschland GmbH) im Handel.

Neben den quaternierten Fettsäuretriethanolaminestersalzen kommen als Esterquats ferner auch quatemierte Estersalze von Fettsäuren mit Diethanolalkylaminen der Formel (II) in Betracht, 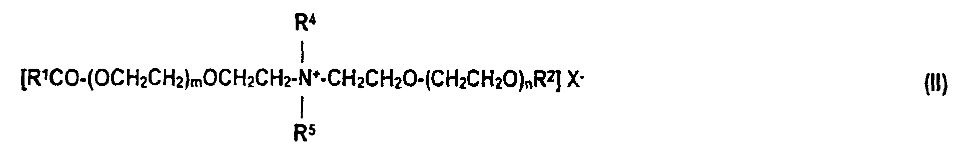 in der R¹CO für einen Acylrest mit 6 bis 22 Kohlenstoffatomen, R² für Wasserstoff oder R¹CO, R⁴ und R⁵ unabhängig voneinander für Alkylreste mit 1 bis 4 Kohlenstoffatomen, m und n in Summe für 0 oder Zahlen von 1 bis 12 und X für Halogenid, Alkylsulfat oder Alkylphosphat steht.

Als weitere Gruppe geeigneter Esterquats sind schließlich die quatemierten Estersalze von Fettsäuren mit 1,2-Dihydroxypropyldiatkylaminen der Formel **(III)** zu nennen, 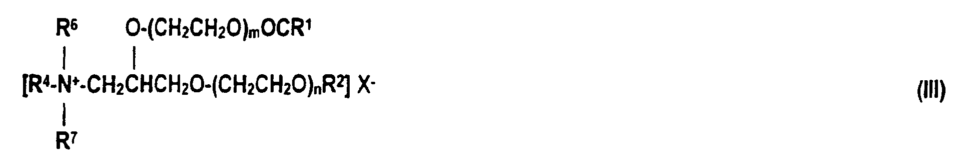 in der R¹CO für einen Acylrest mit 6 bis 22 Kohlenstoffatomen, R² für Wasserstoff oder R¹CO, R⁴, R⁶ und R⁷ unabhängig voneinander für Alkylreste mit 1 bis 4 Kohlenstoffatomen, m und n in Summe für 0 oder Zahlen von 1 bis 12 und X für Halogenid, Alkylsulfat oder Alkylphosphat steht.

Des weiteren kommen als Esterquats noch Stoffe in Frage, bei denen die Ester- durch eine Amidbindung ersetzt ist und die vorzugsweise basierend auf Diethylentriamin der Formel **(IV)** folgen, 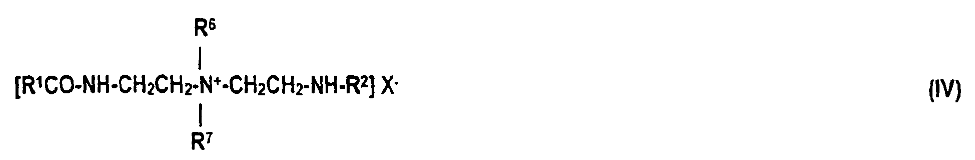 in der R¹CO für einen Acylrest mit 6 bis 22 Kohlenstoffatomen, R² für Wasserstoff oder R¹CO, R⁶ und R⁷ unabhängig voneinander für Alkylreste mit 1 bis 4 Kohlenstoffatomen und X für Halogenid, Alkylsulfat oder Alkylphosphat steht. Derartige Amidesterquats sind beispielsweise unter der Marke Incroquat® (Croda) im Markt erhältlich.

Schließlich kommen als Esterquats auch Stoffe in Frage, die auf Basis von ethoxyliertem Ricinusöl oder dessen Härtungsprodukten erhältlich sind und vorzugsweise der Formel **(V)** folgen, 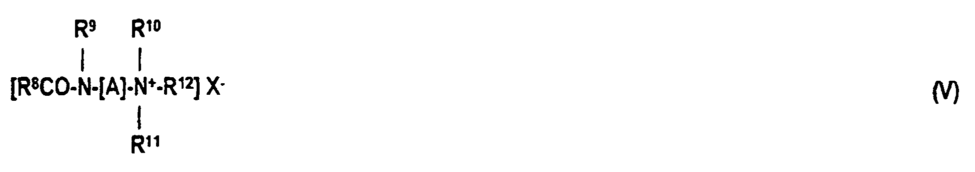 in der R⁸CO für einen gesättigten und/oder ungesättigten ethoxylierten Hydroxyacylrest mit 16 bis 22, vorzugsweise 18 Kohlenstoffatomen sowie 1 bis 50 Oxyethyleneinheiten, A für einen linearen oder verzweigten Alkylenrest mit 1 bis 6 Kohlenstoffatomen, R⁹, R¹⁰ und R¹¹ unabhängig voneinander für Wasserstoff oder eine Alkylgruppe mit 1 bis 4 Kohlenstoffatomen, R¹² für einen Alkylrest mit 1 bis 4 Kohlenstoffatomen oder einen Benzylrest und X für Halogen, Alkylsulfat oder Alkylphosphat steht.

Hinsichtlich der Auswahl der bevorzugten Fettsäuren und des optimalen Veresterungsgrades gelten die für (1) genannten Beispiele auch für die Esterquats der Formeln **(II)** bis **(V)**.

Zur Herstellung der Esterquats der Formeln (1) bis (V) kann sowohl von Fettsäuren als auch den entsprechenden Triglyceriden ausgegangen werden. Ein solches Verfahren, das stellvertretend für den entsprechenden Stand der Technik genannt werden soll, wird in der europäischen Patentschrift **EP 0750606 B1** (Cognis) vorgeschlagen. Ebenfalls ist es möglich, die Kondensation der Alkanolamine mit den Fettsäuren in Gegenwart definierter Mengen an Dicarbonsäuren, wie z.B. Oxalsäure, Malonsäure, Bemsteinsäure, Maleinsäure, Fumarsäure, Glutarsäure, Adipinsäure, Sorbinsäure, Pimelinsäure, Azelainsäure, Sebacinsäure und/oder Dodecandisäure durchzuführen. Auf diese Weise kommt es zur einer partiell oligomeren Struktur der Esterquats, was sich insbesondere bei Mitverwendung von Adipinsäure auf die Klarlöslichkeit der Produkte vorteilhaft auswirken kann. Entsprechende Produkte unter der Marke Dehyquart® D 6003 (Cognis Deutschland GmbH) sind im Handel erhältlich und werden beispielsweise in der Europäischen Patentschrift **EP 0770594 B1** (Cognis) beschrieben. Üblicherweise gelangen die Esterquats in Form 50 bis 90 Gew.-%iger alkoholischer Lösungen in den Handel, die bei Bedarf problemlos mit Wasser verdünnt werden können.

### Tetraalkylammoniumverbindungen

Unter Tetraalkylammoniumverbindungen (quartäre Ammoniumverbindungen, QAV) sind kationische Tenside zu verstehen, bei denen der quartäre Sticktoff von 4 Alkylresten substituiert wird. Die Alkylreste können ihrerseits ebenfalls Substituenten tragen, wie z.B. Hydroxygruppen oder Phenylreste. Ebenfalls kann der quartäre Stickstoff Teil eines naphthenischen Ringsystems sein. Tetraalkylammoniumverbindungen, die im Sinne der Erfindung in Betracht kommen, folgen vorzugsweise der Formel **(VI)**, 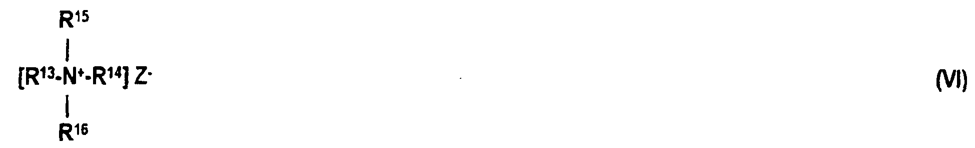 in der R¹³ für einen linearen oder verzweigten, gegebenenfalls hydroxysubstituierten Alkylrest mit 6 bis 22 Kohlenstoffatomen oder einen Benzylrest, R¹⁴ und R¹⁵ unabhängig voneinander für einen gegebenenfalls hydroxysubstituierten Alkylrest mit 1 bis 22 Kohlenstoffatomen, R¹⁶ für einen gegebenenfalls hydroxysubstituierten Alkylrest mit 1 bis 4 Kohlenstoffatomen und Z für Halogenid, Alkylsulfat oder Alkylphosphat steht. Im Hinblick auf die gewünschten anwendungstechnischen Eigenschaften hat es sich als besonders vorteilhaft erwiesen, wenn die Tetraalkylammoniumverbindungen ein oder zwei langkettige und zwei oder drei kurzkettige Substituenten aufweisen, wie dies beispielsweise beim Dimethyldistearylammoniumchlorid der Fall ist. Weitere besonders geeignete Verbindungen sind Cetyltrimethylammoniumchlorid (Dehyquart® A, Cognis Deutschland GmbH/Düsseldorf) und insbesondere Hydroxycetyl Hydroxyethyl Dimoniumchlorid (Dehyquart® E, Cognis Deutschland GmbH/Düsseldorf).
QAV mit zwei langen und zwei kurzen Alkylsubstituenten am Stickstoff finden beispielsweise als Konditioniermittel in der Kosmetik sowie als Avivagemittel in Weichspülern Verwendung. Eine Übersicht zu Herstellung und Verwendung von QAV findet sich beispielsweise von Bell et al. in **INFORM, 7, 992 (1996).**

### Kationpolymere

Geeignete kationische Polymere sind beispielsweise kationische Cellulosederivate, wie z.B. eine quatemierte Hydroxyethylcellulose, die unter der Bezeichnung Polymer JR 400® von Amerchol erhältlich ist, kationische Stärke, Copolymere von Diallylammoniumsalzen und Acrylamiden, quatemierte Vinylpyrrolidon/Vinylimidazol-Polymere, wie z.B. Luviquat® (BASF), Kondensationsprodukte von Polyglycolen und Aminen, quatemierte Kollagenpolypeptide, wie beispielsweise Lauryldimonium hydroxypropyl hydrolyzed collagen (Lamequat®L/Grünau), quaternierte Weizenpolypeptide, Polyethylenimin, kationische Siliconpolymere, wie z.B. Amidomethicone, Copolymere der Adipinsäure und Dimethylaminohydroxypropyldiethylentriamin (Cartaretine®/Sandoz), Copolymere der Acrylsäure mit Dimethyldiallylammoniumchlorid (Merquat® 550/Chemviron), Polyaminopolyamide, wie z.B. beschrieben in der **FR 2252840 A** sowie deren vemetzte wasserlöslichen Polymere, kationische Chitinderivate, wie beispielsweise quatemiertes Chitosan, gegebenenfalls mikrokristallin verteilt, Kondensationsprodukte aus Dihalogenalkylen, wie z.B. Dibrombutan mit Bisdialkylaminen, wie z.B. Bis-Dimethylamino-1,3-propan, kationischer Guar-Gum, wie z.B. Jaguar® CBS, Jaguar® C-17, Jaguar® C-16 der Firma Celanese, quaternierte Ammoniumsalz-Polymere, wie z.B. Mirapol® A-15, Mirapol® AD-1, Mirapol® AZ-1 der Firma Miranol.

### Herstellung von Nanopartikeln

Ein Verfahren zur Herstellung von Nanoteilchen durch rasche Entspannung von überkritischen Lösungen **(Rapid Expansion of Supercritical Solutions RESS)** ist beispielsweise aus dem Aufsatz von S.Chihlar, M.Türk und K.Schaber in **Proceedings World Congress on Particle Technology 3, Brighton, 1998** bekannt. In einer bevorzugten Ausführungsform der Erfindung setzt man nanoskalige kationischen Verbindungen ein, die man erhält, indem man
(a) die Ausgangsstoffe unter überkritischen oder nahekritischen Bedingungen in einem geeigneten Lösungsmittel löst,
(b) die fluide Mischung über eine Düse in ein Vakuum, ein Gas oder eine Flüssigkeit entspannt, und
(c) das Lösemittel dabei gleichzeitig verdampft.

Um zu verhindern, daß die Nanoteilchen wieder zusammenbacken, empfiehlt es sich, die Ausgangsstoffe in Gegenwart geeigneter Schutzkolloide oder Emulgatoren zu lösen und/oder die kritischen Lösungen in wäßrige und/oder alkoholische Lösungen der Schutzkolloide bzw. Emulgatoren oder aber in kosmetische Öle zu entspannen, welche ihrerseits wieder gelöste Emulgatoren und/oder Schutzkolloide enthalten können. Geeignete Schutzkolloide sind dabei z.B. Gelatine, Casein, Gummi arabicum, Lysalbinsäure, Stärke sowie Polymere, wie etwa Polyvinylalkohole, Polyvinylpyrrolidone Polyalkylenglycole und Polyacrylate. Die bevorzugt zu verwendenden nanoskaligen kationischen Verbindungen sind also die, die von einem Schutzkolloid und/oder einem Emulgator ummantelt vorliegen. Üblicherweise werden die Schutzkolloide oder Emulgatoren in Mengen von 0,1 bis 20, vorzugsweise 5 bis 15 Gew.-% - bezogen auf die kationischen Verbindungen - eingesetzt.

Ein weiteres geeignetes Verfahren zur Herstellung der nanoskaligen Teilchen bietet die **Evaporationstechnik**. Hierbei werden die Ausgangsstoffe zunächst in einem geeigneten organischen Lösungsmittel (z.B. Alkane, pflanzliche Öle, Ether, Ester, Ketone, Acetale und dergleichen) gelöst. Anschließend werden die Lösungen derart in Wasser oder einem anderen Nicht-Lösungsmittel, gegebenenfalls in Gegenwart einer darin gelösten oberflächenaktiven Verbindung gegeben, daß es durch die Homogenisierung der beiden nicht miteinander mischbaren Lösungsmittel zu einer Ausfällung der Nanoteilchen kommt, wobei das organische Lösungsmittel vorzugsweise verdampft. Anstelle einer wäßrigen Lösung können auch O/W-Emulsionen bzw. O/W-Mikroemulsionen eingesetzt werden. Als oberflächenaktive Verbindungen können die bereits eingangs erläuterten Emulgatoren und Schutzkolloide verwendet werden. Eine weitere Möglichkeit zur Herstellung von Nanoteilchen besteht in dem sogenannten **GAS-Verfahren** (Gas Anti Solvent Recrystallization). Das Verfahren nutzt ein hochkomprimiertes Gas oder überkritisches Fluid (z.B. Kohlendioxid) als Nicht-Lösungsmittel zur Kristallisation von gelösten Stoffen. Die verdichtete Gasphase wird in die Primärlösung der Ausgangsstoffe eingeleitet und dort absorbiert, wodurch sich das Flüssigkeitsvolumen vergrößert, die Löslichkeit abnimmt und feinteilige Partikel ausgeschieden werden. Ähnlich geeignet ist das **PCA-Verfahren** (Precipitation with a Compressed Fluid Anti-Solvent). Hier wird die Primärlösung der Ausgangsstoffe in ein überkritisches Fluid eingeleitet, wobei sich feinstverteilte Tröpfchen bilden, in denen Diffusionsvorgänge ablaufen, so daß eine Ausfällung feinster Partikel erfolgt. Beim **PGSS-Verfahren** (Particles from Gas Saturated Solutions) werden die Ausgangsstoffe durch Aufpressen von Gas (z.B. Kohlendioxid oder Propan) aufgeschmolzen. Druck und Temperatur erreichen nahe- oder überkritische Bedingungen. Die Gasphase löst sich im Feststoff und bewirkt eine Absenkung der Schmelztemperatur, der Viskosität und der Oberflächenspannung. Bei der Expansion durch eine Düse kommt es durch Abkühlungseffekte zur Bildung feinster Teilchen. Wäscheweichspülmittel als auch für kosmetische Anwendungen zunehmend an Bedeutung gewinnen.

### Gewerbliche Anwendbarkeit

Gegenüber kationischen Verbindungen des Stands der Technik bewirkt die besondere Feinteiligkeit der Partikel eine erhöhte Stabilität und Konsistenz der Emulsionen. In kosmetischen Zubereitungen werden diese Verbindungen zur Erzielung eines angenehmen weichen Haut- und Haargefühls eingesetzt. Sie können zur Herstellung von Emulsionen, Cremes, Gele und Lotionen zur Hautpflege wie auch Shampoos, Duschbädem, Spülungen, Conditionem und dergleichen für die Haarpflege eingesetzt werden. Weiterhin können die erfindungsgemäßen Mittel in Wäscheweichspülern Verwendung finden. Ein weiterer Gegenstand der vorliegenden Erfindung betrifft daher die Verwendung der nanoskaligen kationischen Verbindungen zur Herstellung Wäscheweichspülern. Die Einsatzmenge der kationischen Verbindungen liegt dabei üblicherweise in der Größenordnung von 0,1 bis 10, vorzugsweise 0,5 bis 8 und insbesondere 1 bis 5 Gew.-% - bezogen auf die Zubereitungen.

Die kosmetischen und/oder pharmazeutischen Zubereitungen sowie Wäscheweichspülmittel können femer als weitere Hilfs- und Zusatzstoffe milde Tenside, Ölkörper, Emulgatoren, Überfettungsmittel, Perlglanzwachse, Konsistenzgeber, Verdickungsmittel, Polymere, Siliconverbindungen, Fette, Wachse, biogene Wirkstoffe, Deodorantien, Filmbildner, Quellmittel, Antioxidantien, Hydrotrope, Konservierungsmittel, Solubilisatoren, Parfümöle, Farbstoffe und dergleichen enthalten.

Typische Beispiele für geeignete milde, d.h. besonders hautverträgliche **Tenside** sind Feftalkoholpolyglycolethersulfate, Monoglyceridsulfate, Mono- und/oder Dialkylsulfosuccinate, Fettsäureisethionate, Fettsäuresarcosinate, Fettsäuretauride, Fettsäureglutamate, α-Olefinsulfonate, Ethercarbonsäuren, Alkyloligoglucoside, Fettsäureglucamide, Alkylamidobetaine und/oder Proteinfettsäurekondensate, letztere vorzugsweise auf Basis von Weizenproteinen.

Als **Ölkörper** kommen beispielsweise Guerbetalkohole auf Basis von Fettalkoholen mit 6 bis 18, vorzugsweise 8 bis 10 Kohlenstoffatomen, Ester von linearen C₆-C₂₂-Fettsäuren mit linearen C₆-C₂₂-Fettalkoholen, Ester von verzweigten C₆-C₁₃-Carbonsäuren mit linearen C₆-C₂₂-Fettalkoholen, wie z.B. Myristylmyristat, Myristylpalmitat, Myristylstearat, Myristylisostearat, Myristyloleat, Myristylbehenat, Myristylerucat, Cetylmyristat, Cetylpalmitat, Cetylstearat, Cetylisostearat, Cetyloleat, Cetylbehenat, Cetylerucat, Stearylmyristat, Stearylpalmitat, Stearylstearat, Stearylisostearat, Stearyloleat, Stearylbehenat, Stearylerucat, Isostearylmyristat, Isostearylpalmitat, Isostearylstearat, Isostearylisostearat, Isostearyloleat, Isostearylbehenat, Isostearyloleat, Oleylmyristat, Oleylpalmitat, Oleylstearat, Oleylisostearat, Oleyloleat, Oleylbehenat, Oleylerucat, Behenylmyristat, Behenylpalmitat, Behenylstearat, Behenylisostearat, Behenyloleat, Behenylbehenat, Behenylerucat, Erucylmyristat, Erucylpalmitat, Erucylstearat, Erucylisostearat, Erucyloleat, Erucylbehenat und Erucylerucat. Daneben eignen sich Ester von linearen C₆-C₂₂-Fettsäuren mit verzweigten Alkoholen, insbesondere 2-Ethylhexanol, Ester von Hydroxycarbonsäuren mit linearen oder verzweigten C₆-C₂₂-Fettalkoholen, insbesondere Dioctyl Malate, Ester von linearen und/oder verzweigten Fettsäuren mit mehrwertigen Alkoholen (wie z.B. Propylenglycol, Dimerdiol oder Trimertriol) und/oder Guerbetalkoholen, Triglyceride auf Basis C₆-C₁₀-Fettsäuren, flüssige Mono-/Di-/Triglyceridmischungen auf Basis von C₆-C₁₈-Fettsäuren, Ester von C₆-C₂₂-Fettalkoholen und/oder Guerbetalkoholen mit aromatischen Carbonsäuren, insbesondere Benzoesäure, Ester von C₂-C₁₂-Dicarbonsäuren mit linearen oder verzweigten Alkoholen mit 1 bis 22 Kohlenstoffatomen oder Polyolen mit 2 bis 10 Kohlenstoffatomen und 2 bis 6 Hydroxylgruppen, pflanzliche Öle, verzweigte primäre Alkohole, substituierte Cyclohexane, lineare und verzweigte C₆-C₂₂-Fettalkoholcarbonate, Guerbetcarbonate, Ester der Benzoesäure mit linearen und/oder verzweigten C₆-C₂₂-Alkoholen (z.B. Finsolv® TN), lineare oder verzweigte, symmetrische oder unsymmetrische Dialkylether mit 6 bis 22 Kohlenstoffatomen pro Alkylgruppe, Ringöffnungsprodukte von epoxidierten Fettsäureestem mit Polyolen, Siliconöle und/oder aliphatische bzw. naphthenische Kohlenwasserstoffe, wie z.B. wie Squalan, Squalen oder Dialkylcyclohexane in Betracht.

Als **Emulgatoren** kommen beispielsweise nichtionogene Tenside aus mindestens einer der folgenden Gruppen in Frage:
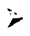 Anlagerungsprodukte von 2 bis 30 Mol Ethylenoxid und/ oder 0 bis 5 Mol Propylenoxid an lineare Fettalkohole mit 8 bis 22 C-Atomen, an Fettsäuren mit 12 bis 22 C-Atomen, an Alkylphenole mit 8 bis 15 C-Atomen in der Alkylgruppe sowie Alkylamine mit 8 bis 22 Kohlenstoffatomen im Alkylrest;
Alkyl- und/oder Alkenyloligoglykoside mit 8 bis 22 Kohlenstoffatomen im Alk(en)ylrest und deren ethoxylierte Analoga;
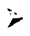 Anlagerungsprodukte von 1 bis 15 Mol Ethylenoxid an Ricinusöl und/oder gehärtetes Ricinusöl;
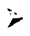 Anlagerungsprodukte von 15 bis 60 Mol Ethylenoxid an Ricinusöl und/oder gehärtetes Ricinusöl;
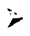 Partialester von Glycerin und/oder Sorbitan mit ungesättigten, linearen oder gesättigten, verzweigten Fettsäuren mit 12 bis 22 Kohlenstoffatomen und/oder Hydroxycarbonsäuren mit 3 bis 18 Kohlenstoffatomen sowie deren Addukte mit 1 bis 30 Mol Ethylenoxid;
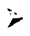 Partialester von Polyglycerin (durchschnittlicher Eigenkondensationsgrad 2 bis 8), Polyethylenglycol (Molekulargewicht 400 bis 5000), Trimethylolpropan, Pentaerythrit, Zuckeralkoholen (z.B. Sor bit), Alkylglucosiden (z.B. Methylglucosid, Butylglucosid, Laurylglucosid) sowie Polyglucosiden (z.B. Cellulose) mit gesättigten und/oder ungesättigten, linearen oder verzweigten Fettsäuren mit 12 bis 22 Kohlenstoffatomen und/oder Hydroxycarbonsäuren mit 3 bis 18 Kohlenstoffatomen sowie deren Addukte mit 1 bis 30 Mol Ethylenoxid;
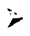 Mischester aus Pentaerythrit, Fettsäuren, Citronensäure und Fettalkohol gemäß **DE 1165574 PS** und/oder Mischester von Fettsäuren mit 6 bis 22 Kohlenstoffatomen, Methylglucose und Polyolen, vorzugsweise Glycerin oder Polyglycerin.
Mono-, Di- und Trialkylphosphate sowie Mono-, Di- und/oder Tri-PEG-alkylphosphate und deren Salze;
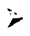 Wollwachsalkohole;
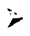 Polysiloxan-Polyalkyl-Polyether-Copolymere bzw. entsprechende Derivate;
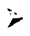 Polyalkylenglycole sowie
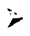 Glycerincarbonat.

Die **Anlagerungsprodukte von Ethylenoxid und/oder von Propylenoxid** an Fettalkohole, Fettsäuren, Alkylphenole oder an Ricinusöl stellen bekannte, im Handel erhältliche Produkte dar. Es handelt sich dabei um Homologengemische, deren mittlerer Alkoxylierungsgrad dem Verhältnis der Stoffmengen von Ethylenoxid und/ oder Propylenoxid und Substrat, mit denen die Anlagerungsreaktion durchgeführt wird, entspricht. C_{12/18}-Fettsäuremono- und -diester von Anlagerungsprodukten von Ethylenoxid an Glycerin sind aus **DE 2024051 PS** als Rückfettungsmittel für kosmetische Zubereitungen bekannt.

**Alkyl- und/oder Alkenyloligoglycoside,** ihre Herstellung und ihre Verwendung sind aus dem Stand der Technik bekannt. Ihre Herstellung erfolgt insbesondere durch Umsetzung von Glucose oder Oligosacchariden mit primären Alkoholen mit 8 bis 18 Kohlenstoffatomen. Bezüglich des Glycosidrestes gilt, daß sowohl Monoglycoside, bei denen ein cyclischer Zuckerrest glycosidisch an den Fettalkohol gebunden ist, als auch oligomere Glycoside mit einem Oligomerisationsgrad bis vorzugsweise etwa 8 geeignet sind. Der Oligomerisierungsgrad ist dabei ein statistischer Mittelwert, dem eine für solche technischen Produkte übliche Homologenverteilung zugrunde liegt.

Typische Beispiele für geeignete **Partialglyceride** sind Hydroxystearinsäuremonoglycerid, Hydroxystearinsäurediglycerid, Isostearinsäuremonoglycerid, Isostearinsäurediglycerid, Ölsäuremonoglycerid, Ölsäurediglycerid, Ricinolsäuremoglycerid, Ricinolsäurediglycerid, Linolsäuremonoglycerid, Linolsäurediglycerid, Linolensäuremonoglycerid, Linolensäurediglycerid, Erucasäuremonoglycerid, Eiucasäurediglycerid, Weinsäuremonoglycerid, Weinsäurediglycerid, Citronensäuremonoglycerid, Citronendiglycerid, Äpfelsäuremonoglycerid, Äpfelsäurediglycerid sowie deren technische Gemische, die untergeordnet aus dem Herstellungsprozeß noch geringe Mengen an Triglycerid enthalten können. Ebenfalls geeignet sind Anlagerungsprodukte von 1 bis 30, vorzugsweise 5 bis 10 Mol Ethylenoxid an die genannten Partialglyceride.

Als **Sorbitanester** kommen Sorbitanmonoisostearat, Sorbitansesquiisostearat, Sorbitandüsostearat, Sorbitantrüsostearat, Sorbitanmonooleat, Sorbitansesquioleat, Sorbitandioleat, Sorbitantrioleat, Sorbitanmonoerucat, Sorbitansesquierucat, Sorbitandierucat, Sorbitantrierucat, Sorbitanmonoricinoleat, Sorbitansesquiricinoleat, Sorbitandiricinoleat, Sorbitantriricinoleat, Sorbitanmonohydroxystearat, Sorbitansesquihydroxystearat, Sorbitandihydroxystearat, Sorbitantrihydroxystearat, Sorbitanmonotartrat, Sorbitansesquitartrat, Sorbitanditartrat, Sorbitantntartrat, Sorbitanmonocitrat, Sorbitansesquicitrat, Sorbitandicitrat, Sorbitantricitrat, Sorbitanmonomaleat, Sorbitansesquimaleat, Sorbitandimaleat, Sorbitantrimaleat sowie deren technische Gemische. Ebenfalls geeignet sind Anlagerungsprodukte von 1 bis 30, vorzugsweise 5 bis 10 Mol Ethylenoxid an die genannten Sorbitanester.

Typische Beispiele für geeignete **Polyglycerinester** sind Polyglyceryl-2 Dipolyhydroxystearate (Dehymuls® PGPH), Polyglycerin-3-Diisostearate (Lameform® TGI), Polyglyceryl-4 Isostearate (Isolan® Gl 34), Polyglyceryl-3 Oleate, Diisostearoyl Polyglyceryl-3 Diisostearate (Isotan® PDI), Polyglyceryl-3 Methylglucose Distearate (Tego Care® 450), Polyglyceryl-3 Beeswax (Cera Bellina®), Polyglyceryl-4 Caprate (Polyglycerol Caprate T2010/90), Polyglyceryl-3 Cetyl Ether (Chimexane® NL), Polyglyceryl-3 Distearate (Cremophor® GS 32) und Polyglyceryl Polyricinoleate (Admul® WOL 1403) Polyglyceryl Dimerate Isostearate sowie deren Gemische.
Beispiele für weitere geeignete **Polyolester** sind die gegebenenfalls mit 1 bis 30 Mol Ethylenoxid umgesetzten Mono-, Di- und Triester von Trimethylolpropan oder Pentaerythrit mit Laurinsäure, Kokosfettsäure, Talgfettsäure, Palmitinsäure, Stearinsäure, Ölsäure, Behensäure und dergleichen.

Weiterhin können als Emulgatoren **zwitterionische Tenside** verwendet werden. Als zwitterionische Tenside werden solche oberflächenaktiven Verbindungen bezeichnet, die im Molekül mindestens eine quartäre Ammoniumgruppe und mindestens eine Carboxylat- und eine Sulfonatgruppe tragen. Besonders geeignete zwitterionische Tenside sind die sogenannten Betaine wie die N-Alkyl-N,N-dimethylammoniumglycinate, beispielsweise das Kokosalkyldimethylammoniumglycinat, N-Acylaminopropyl-N,N-dimethylammoniumglycinate, beispielsweise das Kokosacylaminopropyldimethylammoniumglycinat, und 2-Afkyf-3-carboxylmethyf-3-hydroxyethyümidazoüne mit jeweils 8 bis 18 C-Atomen in der Alkyl- oder Acylgruppe sowie das Kokosacylaminoethylhydroxyethylcarboxymethylglycinat. Besonders bevorzugt ist das unter der CTFA-Bezeichnung *Cocamidopropyl Betaine* bekannte Fettsäureamid-Derivat. Ebenfalls geeignete Emulgatoren sind ampholytische Tenside. Unter ampholytischen Tensiden werden solche oberflächenaktiven Verbindungen verstanden, die außer einer C_{8/18}-Alkyl- oder -Acylgruppe im Molekül mindestens eine freie Aminogruppe und mindestens eine -COOH- oder -SO₃H-Gruppe enthalten und zur Ausbildung innerer Salze befähigt sind. Beispiele für geeignete ampholytische Tenside sind N-Alkylglycine, N-Alkylpropionsäuren, N-Alkylaminobuttersäuren, N-Alkyliminodipropionsäuren, N-Hydroxyethyl-N-alkylamidopropylglycine, N-Alkyltaurine, N-Alkylsarcosine, 2-Alkylaminopropionsäuren und Alkylaminoessigsäuren mit jeweils etwa 8 bis 18 C-Atomen in der Alkylgruppe. Besonders bevorzugte ampholytische Tenside sind das N-Kokosalkylaminopropionat, das Kokosacylaminoethylaminopropionat und das C_{12/18}-Acylsarcosin.

Als **Überfettungsmittel** können Substanzen wie beispielsweise Lanolin und Lecithin sowie polyethoxylierte oder acylierte Lanolin- und Lecithinderivate, Polyolfeftsäureester, Monoglyceride und Fettsäurealkanolamide verwendet werden, wobei die letzteren gleichzeitig als Schaumstabilisatoren dienen.

Als **Perlglanzwachse** kommen beispielsweise in Frage: Alkylenglycolester, speziell Ethylenglycoldistearat; Fettsäurealkanolamide, speziell Kokosfettsäurediethanolamid; Partialglyceride, speziell Stearinsäuremonoglycerid; Ester von mehrwertigen, gegebenenfalls hydroxysubstituierte Carbonsäuren mit Fettalkoholen mit 6 bis 22 Kohlenstoffatomen, speziell langkettige Ester der Weinsäure; Fettstoffe, wie beispielsweise Fettalkohole, Fettketone, Fettaldehyde, Fettether und Fettcarbonate, die in Summe mindestens 24 Kohlenstoffatome aufweisen, speziell Lauron und Distearylether; Fettsäuren wie Stearinsäure, Hydroxystearinsäure oder Behensäure, Ringöffnungsprodukte von Olefinepoxiden mit 12 bis 22 Kohlenstoffatomen mit Fettalkoholen mit 12 bis 22 Kohlenstoffatomen und/oder Polyolen mit 2 bis 15 Kohlenstoffatomen und 2 bis 10 Hydroxylgruppen sowie deren Mischungen.

Als **Konsistenzgeber** kommen in erster Linie Fettalkohole oder Hydroxytettalkohole mit 12 bis 22 und vorzugsweise 16 bis 18 Kohlenstoffatomen und daneben Partialglyceride, Fettsäuren oder Hydroxyfettsäuren in Betracht. Bevorzugt ist eine Kombination dieser Stoffe mit Alkyloligoglucosiden und/oder Fettsäure-N-methylglucamiden gleicher Kettenlänge und/oder Polyglycerinpoly-12-hydroxystearaten.

Geeignete **Verdickungsmittel** sind beispielsweise Aerosil-Typen (hydrophile Kieselsäuren), Polysaccharide, insbesondere Xanthan-Gum, Guar-Guar, Agar-Agar, Alginate und Tylosen, Carboxymethylcellulose und Hydroxyethylcellulose, femer höhermolekulare Polyethylenglycolmono- und -diester von Fettsäuren, Polyacrylate, (z.B. Carbopole® von Goodrich oder Synthalene® von Sigma), Polyacrylamide, Polyvinylalkohol und Polyvinylpyrrolidon, Tenside wie beispielsweise ethoxylierte Fettsäureglyceride, Ester von Fettsäuren mit Polyolen wie beispielsweise Pentaerythrit oder Trimethylolpropan, Fettalkoholethoxylate mit eingeengter Homologenverteilung oder Alkyloligoglucoside sowie Elektrolyte wie Kochsalz und Ammoniumchlorid.

Als **anionische, zwitterionische, amphotere und nichtionische Polymere** kommen beispielsweise Vinylacetat/Crotonsäure-Copolymere, Vinylpyrrolidon/Vinylacrylat-Copolymere, Vinylacetat/Butylmaleat/Isobornylacrylat-Copolymere, Methylvinylether/Maleinsäureanhydrid-Copolymere und deren Ester, unvemetzte und mit Polyolen vemetzte Polyacrylsäuren, Acrylamidopropyltrimethylammoniumchlorid/Acrylat-Copolymere, Octylacrylamid/Methylmethacrylat/tert.Butylaminoethylmethacrylat/2-Hydroxyproylmethacrylat-Copolymere, Polyvinylpyrrolidon, Vinylpyrrolidon/Vinylacetat-Copolymere, Vinylpyrrolidon/Dimethylaminoethylmethacrylat/Vinylcaprolactam-Terpolymere sowie gegebenenfalls derivatisierte Celluloseether und Silicone in Frage.

Geeignete Siliconverbindungen sind beispielsweise Dimethylpolysiloxane, Methylphenylpolysiloxane, cyclische Silicone sowie amino-, fettsäure-, alkohol-, polyether-, epoxy-, fluor-, glykosid- und/oder alkylmodifizierte Siliconverbindungen, die bei Raumtemperatur sowohl flüssig als auch harzförmig vorliegen können. Weiterhin geeignet sind Simethicone, bei denen es sich um Mischungen aus Dimethiconen mit einer durchschnittlichen Kettenlänge von 200 bis 300 Dimethylsiloxan-Einheiten und hydrierten Silicaten handelt. Eine detaillierte Übersicht über geeignete flüchtige Silicone findet sich zudem von Todd et al. in **Cosm.Toil. 91, 27 (1976)**.

Typische Beispiele für **Fette** sind Glyceride, als **Wachse** kommen u.a. natürliche Wachse, wie z.B. Candelillawachs, Camaubawachs, Japanwachs, Espartograswachs, Korkwachs, Guarumawachs, Reiskeimölwachs, Zuckerrohrwachs, Ouricurywachs, Montanwachs, Bienenwachs, Schellackwachs, Walrat, Lanolin (Wollwachs), Bürzelfett, Ceresin, Ozokerit (Erdwachs), Petrolatum, Paraffinwachse, Mikrowachse; chemisch modifizierte Wachse (Hartwachse), wie z.B. Montanesterwachse, Sasolwachse, hydrierte Jojobawachse sowie synthetische Wachse, wie z.B. Polyalkylenwachse und Polyethylenglycolwachse in Frage.
Unter **biogenen Wirkstoffen** sind beispielsweise Tocopherol, Tocopherolacetat, Tocopherolpalmitat, Ascorbinsäure, Desoxyribonucleinsäure, Retinol, Bisabolol, Allantoin, Phytantriol, Panthenol, AHA-Säuren, Aminosäuren, Ceramide, Pseudoceramide, essentielle Öle, Pflanzenextrakte und Vitaminkomplexe zu verstehen.

Kosmetische **Deodorantien** (Desodorantien) wirken Körpergerüchen entgegen, überdecken oder beseitigen sie. Körpergerüche entstehen durch die Einwirkung von Hautbakterien auf apokrinen Schweiß, wobei unangenehm riechende Abbauprodukte gebildet werden. Dementsprechend enthalten Deodorantien Wirkstoffe, die als keimhemmende Mittel, Enzyminhibitoren, Geruchsabsorber oder Geruchsüberdecker sowie Antitranspirantien fungieren.

Als **keimhemmende Mittel** sind grundsätzlich alle gegen grampositive Bakterien wirksamen Stoffe geeignet, wie z. B. 4-Hydroxybenzoesäure und ihre Salze und Ester, N-(4-Chlorphenyl)-N'-(3,4 dichlor phenyl)hamstoff, 2,4,4'-Trichlor-2'-hydroxydiphenylether (Triclosan), 4-Chlor-3,5-dimethylphenol, 2,2'-Methylen-bis(6-brom-4-chlorphenol), 3-Methyl-4-(1-methylethyl)phenol, 2-Benzyl-4-chlorphenol, 3-(4-Chlorphenoxy)-1,2-propandiol, 3-lod-2-propinylbutylcarbamat, Chlorhexidin, 3,4,4'-Trichlorcarbanilid (TTC), antibakterielle Riechstoffe, Thymol, Thymianöl, Eugenol, Nelkenöl, Menthol, Minzöl, Famesol, Phenoxyethanol, Glycerinmonolaurat (GML), Diglycerinmonocaprinat (DMC), Salicylsäure-N-alkylamide wie z. B. Salicylsäure-n-octylamid oder Salicylsäure-n-decylamid.

Als **Enzyminhibitoren** sind beispielsweise Esteraseinhibitoren geeignet. Hierbei handelt es sich vorzugsweise um Trialkylcitrate wie Trimethylcitrat, Tripropylcitrat, Triisopropylcitrat, Tributylcitrat und insbesondere Triethylcitrat (Hydagen® CAT, Cognis GmbH, Düsseldorf/FRG). Die Stoffe inhibieren die Enzymaktivität und reduzieren dadurch die Geruchsbildung. Weitere Stoffe, die als Esteraseinhibitoren in Betracht kommen, sind Sterolsulfate oder -phosphate, wie beispielsweise Lanosterin-, Cholesterin-, Campesterin-, Stigmasterin- und Sitosterinsulfat bzw -phosphat, Dicarbonsäuren und deren Ester, wie beispielsweise Glutarsäure, Glutarsäuremonoethylester, Glutarsäurediethylester, Adipinsäure. Adipinsäuremonoethylester, Adipinsäurediethylester, Malonsäure und Malonsäurediethylester, Hydroxycarbnonsäuren und deren Ester wie beispielsweise Citronensäure, Äpfelsäure, Weinsäure oder Weinsäurediethylester, sowie Zinkglycinat.

Als **Geruchsabsorber** eignen sich Stoffe, die geruchsbildende Verbindungen aufnehmen und weitgehend festhalten können. Sie senken den Partialdruck der einzelnen Komponenten und verringern so auch ihre Ausbreitungsgeschwindigkeit. Wichtig ist, daß dabei Parfums unbeeinträchtigt bleiben müssen. Geruchsabsorber haben keine Wirksamkeit gegen Bakterien. Sie enthalten beispielsweise als Hauptbestandteil ein komplexes Zinksalz der Ricinolsäure oder spezielle, weitgehend geruchs neutrale Duftstoffe, die dem Fachmann als "Fixateure" bekannt sind, wie z. B. Extrakte von Labdanum bzw. Styrax oder bestimmte Abietinsäurederivate. Als Geruchsüberdecker fungieren Riechstoffe oder Parfümöle, die zusätzlich zu ihrer Funktion als Geruchsüberdecker den Deodorantien ihre jeweilige Duftnote verleihen. Als Parfümöle seien beispielsweise genannt Gemische aus natürlichen und synthetischen Riechstoffen. Natürliche Riechstoffe sind Extrakte von Blüten, Stengeln und Blättern, Früchten, Fruchtschalen, Wurzeln, Hölzern, Kräutern und Gräsern, Nadeln und Zweigen sowie Harzen und Balsamen. Weiterhin kommen tierische Rohstoffe in Frage, wie beispielsweise Zibet und Castoreum. Typische synthetische Riechstoffverbindungen sind Produkte vom Typ der Ester, Ether, Aldehyde, Ketone, Alkohole und Kohlenwasserstoffe. Riechstoffverbindungen vom Typ der Ester sind z.B. Benzylacetat, p-tert.-Butylcyclohexylacetat, Linalylacetat, Phenylethylacetat, Linalylbenzoat, Benrylformiat, Allylcyclohexylpropionat, Styrallylpropionat und Benzylsalicylat. Zu den Ethern zählen beispielsweise Benzylethylether, zu den Aldehyden z.B. die linearen Alkanale mit 8 bis 18 Kohlenstoffatomen, Citral, Citronellal, Citronellyloxyacetaldehyd, Cyclamenaldehyd, Hydroxycitronellal, Lilial und Bourgeonal, zu den Ketonen z.B. die Jonone und Methylcedrylketon, zu den Alkoholen Anethol, Citronellol, Eugenol, Isoeugenol, Geraniol, Linalool, Phenylethylalkohol und Terpineol, zu den Kohlenwasserstoffen gehören hauptsächlich die Terpene und Balsame. Bevorzugt werden jedoch Mischungen verschiedener Riechstoffe verwendet, die gemeinsam eine ansprechende Duftnote erzeugen. Auch ätherische Öle geringerer Flüchtigkeit, die meist als Aromakomponenten verwendet werden, eignen sich als Parfümöle, z.B. Salbeiöl, Kamillenöl, Nelkenöl, Melissenöl, Minzenöl, Zimtblätteröl, Lindenblütenöl, Wacholderbeerenöl, Vetiveröl, Olibanöl, Galbanumöl, Labdanumöl und Lavandinöl. Vorzugsweise werden Bergamotteöl, Dihydromyrcenol, Lilial, Lyral, Citronellol, Phenylethylalkohol, α-Hexylzimtaldehyd, Geraniol, Benzylaceton, Cyclamenaldehyd, Linalool, Boisambrene Forte, Ambroxan, Indol, Hedione, Sandelice, Citronenöl, Mandarinenöl, Orangenöl, Allylamylglycolat, Cyclovertal, Lavandinöl, Muskateller Salbeiöl, β-Damascone, Geraniumöl Bourbon, Cyclohexylsalicylat, Vertofix Coeur, lso-E-Super, Fixolide NP, Evernyl, lraldein gamma, Phenylessigsäure, Geranylacetat, Benzylacetat, Rosenoxid, Romilat, Irotyl und Floramat allein oder in Mischungen, eingesetzt.

**Antitranspirantien** (Antiperspirantien) reduzieren durch Beeinflussung der Aktivität der ekkrinen Schweißdrüsen die Schweißbildung, und wirken somit Achselnässe und Körpergeruch entgegen. Wässrige oder wasserfreie Formulierungen von Antitranspirantien enthalten typischerweise folgende Inhaltsstoffe:
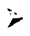 adstringierende Wirkstoffe,
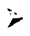 Ölkomponenten,
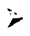 nichtionische Emulgatoren,
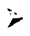 Coemulgatoren,
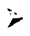 Konsistenzgeber,
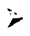 Hilfsstoffe wie z. B. Verdicker oder Komplexierungsmittel und/oder
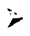 nichtwässrige Lösungsmittel wie z. B. Ethanol, Propylenglykol und/oder Glycerin.

Als adstringierende Antitranspirant-Wirkstoffe eignen sich vor allem Salze des Aluminiums, Zirkoniums oder des Zinks. Solche geeigneten antihydrotisch wirksamen Wirkstoffe sind z.B. Aluminiumchlorid, Aluminiumchlorhydrat, Aluminiumdichlorhydrat, Aluminiumsesquichlorhydrat und deren Komplexverbindungen z. B. mit Propylenglycol-1,2. Aluminiumhydroxyallantoinat, Aluminiumchloridtartrat, Aluminium-Zirkonium-Trichlorohydrat, Aluminium-Zirkonium-tetrachlorohydrat, Aluminium-Zirkonium-pentachlorohydrat und deren Komplexverbindungen z. B. mit Aminosäuren wie Glycin. Daneben können in Antitranspirantien übliche öllösliche und wasserlösliche Hilfsmittel in geringeren Mengen enthalten sein. Solche öllöslichen Hilfsmittel können z.B. sein:
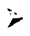 entzündungshemmende, hautschützende oder wohlriechende ätherische Öle,
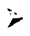 synthetische hautschützende Wirkstoffe und/oder
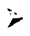 öllösliche Parfümöle.

Übliche wasserlösliche Zusätze sind z.B. Konservierungsmittel, wassedösliche Duftstoffe, pH-Wert-Stellmittel, z.B. Puffergemische, wasserlösliche Verdickungsmittel, z.B. wasserlösliche natürliche oder synthetische Polymere wie z.B. Xanthan-Gum. Hydroxyethylcellulose, Polyvinylpyrrolidon oder hochmolekulare Polyethylenoxide.

Als **Quellmittel** für wäßrige Phasen können Montmorillonite, Clay Mineralstoffe, Pemulen sowie alkylmodifizierte Carbopoltypen (Goodrich) dienen. Weitere geeignete Polymere bzw. Quellmittel können der Übersicht von R.Lochhead in **Cosm.Toil. 108, 95 (1993)** entnommen werden.

Zur Verbesserung des Fließverhaltens können femer Hydrotrope, wie beispielsweise Ethanol, Isopropylalkohol, oder Polyole eingesetzt werden. Polyole, die hier in Betracht kommen, besitzen vorzugsweise 2 bis 15 Kohlenstoffatome und mindestens zwei Hydroxylgruppen. Die Polyole können noch weitere funktionelle Gruppen, insbesondere Aminogruppen, enthalten bzw. mit Stickstoff modifiziert sein. Typische Beispiele sind
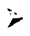 Glycerin;
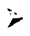 Alkylenglycole, wie beispielsweise Ethylenglycol, Diethylenglycol, Propylenglycol, Butylenglycol, Hexylenglycol sowie Polyethylenglycole mit einem durchschnittlichen Molekulargewicht von 100 bis 1.000 Dalton;
> technische Oligoglyceringemische mit einem Eigenkondensationsgrad von 1,5 bis 10 wie etwa technische Diglyceringemische mit einem Diglyceringehalt von 40 bis 50 Gew.-%;
> Methyolverbindungen, wie insbesondere Trimethylolethan, Trimethylolpropan, Trimethylolbutan, Pentaerythrit und Dipentaerythrit;
> Niedrigalkylglucoside, insbesondere solche mit 1 bis 8 Kohlenstoffen im Alkylrest, wie beispielsweise Methyl- und Butylgtucosid;
> Zuckeralkohole mit 5 bis 12 Kohlenstoffatomen, wie beispielsweise Sorbit oder Mannit,
> Zucker mit 5 bis 12 Kohlenstoffatomen, wie beispielsweise Glucose oder Saccharose;
> Aminozucker, wie beispielsweise Glucamin;
> Dialkoholamine, wie Diethanolamin oder 2-Amino-1,3-propandiol.

Als **Konservierungsmittel** eignen sich beispielsweise Phenoxyethanol, Formaldehydlösung, Parabene, Pentandiol oder Sorbinsäure sowie die in Anlage 6, Teil A und B der Kosmetikverordnung aufgeführten weiteren Stoffklassen.

Als **Parfümöle** seien genannt Gemische aus natürlichen und synthetischen Riechstoffen. Natürliche Riechstoffe sind Extrakte von Blüten (Lilie, Lavendel, Rosen, Jasmin, Neroli, Ylang-Ylang), Stengeln und Blättem (Geranium, Patchouli, Petitgrain), Früchten (Anis, Koriander, Kümmel, Wacholder), Fruchtschalen (Bergamotte, Zitrone, Orangen), Wurzeln (Macis, Angelica, Sellerie, Kardamon, Costus, Iris, Calmus), Hölzern (Pinien-, Sandel-, Guajak-, Zedem-, Rosenholz), Kräutern und Gräsern (Estragon, Lemongras, Salbei, Thymian), Nadeln und Zweigen (Fichte, Tanne, Kiefer, Latschen), Harzen und Balsamen (Galbanum, Elemi, Benzoe, Myrrhe, Olibanum, Opoponax). Weiterhin kommen tierische Rohstoffe in Frage, wie beispielsweise Zibet und Castoreum. Typische synthetische Riechstoffverbindungen sind Produkte vom Typ der Ester, Ether, Aldehyde, Ketone, Alkohole und Kohlenwasserstoffe. Riechstoffverbindungen vom Typ der Ester sind z.B. Benzylacetat, Phenoxyethylisobutyrat, p-tert.-Butylcyclohexylacetat, Linatylacetat, Dimethylbenzylcarbinylacetat, Phenylethylacetat, Linalylbenzoat, Benzylformiat, Ethylmethylphenylglycinat, Allylcyclohexylpropionat, Styrallylpropionat und Benzylsalicylat. Zu den Ethern zählen beispielsweise Benzylethylether, zu den Aldehyden z.B. die linearen Alkanale mit 8 bis 18 Kohlenstoffatomen, Citral, Citronellal, Citronellyloxyacetaldehyd, Cyclamenaldehyd, Hydroxycitronellal, Lilial und Bourgeonal, zu den Ketonen z.B. die Jonone, ∝-Isomethylionon und Methylcedrylketon, zu den Alkoholen Anethol, Citronellol, Eugenol, Isoeugenol, Geraniol, Linalool, Phenylethylalkohol und Terpineol, zu den Kohlenwasserstoffen gehören hauptsächlich die Terpene und Balsame. Bevorzugt werden jedoch Mischungen verschiedener Riechstoffe verwendet, die gemeinsam eine ansprechende Duftnote erzeugen. Auch ätherische Öle geringerer Flüchtigkeit, die meist als Aromakomponenten verwendet werden, eignen sich als Parfümöle, z.B. Salbeiöl, Kamillenöl, Nelkenöl, Melissenöl, Minzenöl, Zimtblätteröl, Lindenblütenöl, Wacholderbeerenöl, Vetiveröl, Olibanöl, Galbanumöl, Labolanumöl und Lavandinöl. Vorzugsweise werden Bergamotteöl, Dihydromyrcenol, Lilial, Lyral, Citronellol, Phenylethylalkohol, α-Hexylzimtaldehyd, Geraniol, Benzylaceton, Cyclamenaldehyd, Linalool, Boisambrene Forte, Ambroxan, Indol, Hedione, Sandelice, Citronenöl, Mandarinenöl, Orangenöl, Allylamylglycolat, Cyclovertal, Lavandinöl, Muskateller Salbeiöl, β-Damascone, Geraniumöl Bourbon, Cyclohexylsalicylat, Vertofix Coeur, Iso-E-Super, Fixolide NP, Evernyl, Iraldein gamma, Phenylessigsäure, Geranylacetat, Benzylacetat, Rosenoxid, Romilllat, Irotyl und Floramat allein oder in Mischungen, eingesetzt.

Als Farbstoffe können die für kosmetische Zwecke geeigneten und zugelassenen Substanzen verwendet werden, wie sie beispielsweise in der Publikation **"Kosmetische Färbemittel" der Farbstoffkommission der Deutschen Forschungsgemeinschaft, Verlag Chemie, Weinheim, 1984, S.81-106** zusammengestellt sind. Diese Farbstoffe werden üblicherweise in Konzentrationen von 0,001 bis 0,1 Gew.-%, bezogen auf die gesamte Mischung, eingesetzt.

Der Gesamtanteil der Hilfs- und Zusatzstoffe kann 1 bis 50, vorzugsweise 5 bis 40 Gew.-% - bezogen auf die Mittel - betragen. Die Herstellung der Mittel kann durch übliche Kalt - oder Heißprozesse erfolgen; vorzugsweise arbeitet man nach der Phaseninversionstemperatur-Methode.

### Beispiele

Zur Herstellung der nanoskaligen Metallseifen (Beispiele 1 bis 5) wurde zunächst Kohlendioxid einem Reservoir mit einem konstanten Druck von 60 bar entnommen und über eine Kolonne mit einer Aktivkohle- und einer Molekularsieb-Packung gereinigt. Nach der Verflüssigung wurde das CO₂ mit Hilfe einer Diaphragma-Pumpe bei einer konstanten Fördermenge von 3,5 l/h auf den gewünschten überkritischen Druck p verdichtet. Anschließend wurde das Lösungsmittel in einem Vorheizer auf die erforderliche Temperatur T1 gebracht und in eine Extraktionskolonne (Stahl, 400 ml) geleitet, welche mit den Wachsen beladen war. Die resultierende überkritische, d.h. fluide Mischung wurde über eine lasergezogene Düse (Länge 830 µm, Durchmesser 45 µm) bei einer Temperatur T2 in eine Plexiglas Expansionskammer versprüht, die eine 4 Gew.%ige wäßrige Lösung eines Emulgators bzw. Schutzkolloids enthielt. Das fluide Medium verdampfte und zurück blieben die im Schutzkolloid eingeschlossenen, dispergierten Nanopartikel. Die Verfahrensbedingungen und der mittlere Partikeigrößenbereich (photometrisch nach der 3-WEM-Methode bestimmt) sind in der nachfolgenden Tabelle 1 angegeben.

**Tabelle 1 Nanopartikel**

| **Bsp.** | **Kationische Verbindungen** | **Lsgm** | **p bar** | **T1 °C** | **T2 °C** | **Emulgator/Schutzkolloid** | **PGB nm** |
|---|---|---|---|---|---|---|---|
| 1 | **Dehyquart® F 75** Distearoylethyl Hydroxyethylmonium Methosulfate + Cetearyl Alco hol | CO₂ | 200 | 80 | 175 | Polyvinylalkohol | 60-120 |
| 2 | **Dehyquart® A-CA** Cetrimonium Chloride | CO₂ | 180 | 70 | 160 | Polyethylenglycol (M=400) | 75-120 |
| 3 | **Jaguar® C-17** Guar gum 2-hydroxypropylether | CO₂ | 200 | 85 | 180 | Polyvinylalkohol | 75-130 |
| 4 | **Lamequat®L** Lauryldimonium hydroxypropyl hydrolyzed collagen | CO₂ | 200 | 85 | 175 | Polyvinylalkohol | 60-140 |
| 5 | **Gluadin® WQ** Kationisches Weizenproteinhydrolysat | CO₂ | 200 | 85 | 175 | Polyvinylalkohol | 55-140 |

Zur Untersuchung der Haaravivage wurden Haarsträhnen vor der Nullmessung mediumblondiert. Die Trockenkämmbarkeit wurde unter Zulassung der elektrostatischen Aufladung untersucht, nach einer Einwirkzeit von 5 min wurden die Testlösungen (1g/1g Haar) unter Standardbedingungen (38 °C, 1l/min) 1 min gespült. Die Messung wurde an 20 Haarsträhnen durchgeführt. Eine ausführliche Beschreibung der Meßmethoden befindet sich in **J.Soc.Cosm.Chem., 24, 782 (1973)**. Die Ergebnisse sind in Tabelle 2 zusammengefaßt. Angegeben ist jeweils die Restarbeit bzw. Restaufladung bezogen auf den Ausgangswert. Die Beurteilung des Haarglanzes erfolgte auf einer Skala von 1 bis 5. Die Beispiele 1 bis 4 sind erfindungsgemäß, die Versuche V1 und V2 dienen zum Vergleich.

**Tabelle 2: Nanoskalige Zubereitungen - Mengenangaben in Gew.-% -**

| **Zusammensetzung/Performance** | **1** | **2** | **3** | **4** | **V1** | **V2** |
|---|---|---|---|---|---|---|
| Sodium Laureth Sulfate | 5,5 | 5,5 | 5,5 | 5,5 | 5,5 | 5,5 |
| Ammonium Laureth Sulfate | 2,4 | 2,4 | 2,4 | 2,4 | 2,4 | 2,4 |
| Cocamide DEA | 1,5 | 1,5 | 1.5 | 1,5 | 1,5 | 1,5 |
| Dimethicone | - | 3 | 3 | 3 | - | 2 |
| **Dehyquart® F 75** Methosulfate + Cetearyl Alcohol | - | - | - | - | 0,3 | - |
| **Gluadin® WQ** Kationisches Weizenproteinhydrolysat | - | - | - | - | - | 2,5 |
| Laureth-2 | 2 | - | 2 | 2 | 2 | 2 |
| Sodium Chloride | 0,2 | 0,3 | 1,0 | 0,5 | 0,5 | 0,5 |
| nanoskalieges Polymer 1 gemäß Tab. 1 | 0,3 | 1 | - | 4 | - | - |
| nanoskalieges Polymer 5 gemäß Tab. 1 | - | 2 | 2 | 0,3 | - | - |
| **Wasser** | ad 100 | | | | | |
| ***Rest-Naßkämmarbeit [%]*** | 62 | 63 | 54 | 54 | 89 | 94 |
| ***Rest-Trockenkämmarbeit [%]*** | 65 | 65 | 67 | 60 | 106 | 77 |
| ***Rest-Aufladung [%]*** | 65 | 59 | 56 | 72 | 110 | 76 |
| ***Stabilität*** | ++ | ++ | + | ++ | + | - |
| ***Dermatologische Verträglichkeit*** | ++ | ++ | + | ++ | + | + |

Die nachfolgende Tabelle 3 enthält eine Reihe von Formulierungsbeispielen mit kationischen Nanopartikeln.

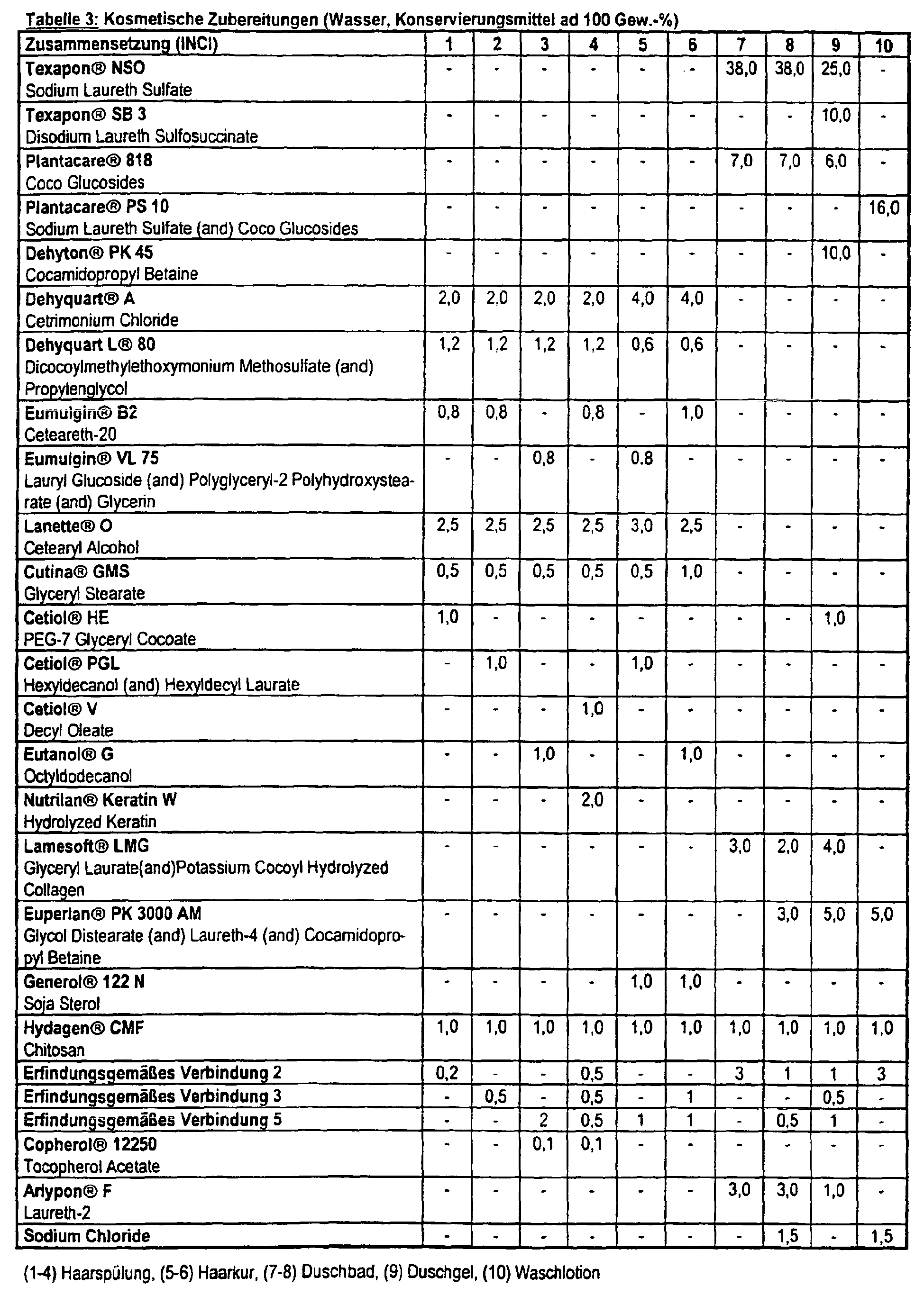

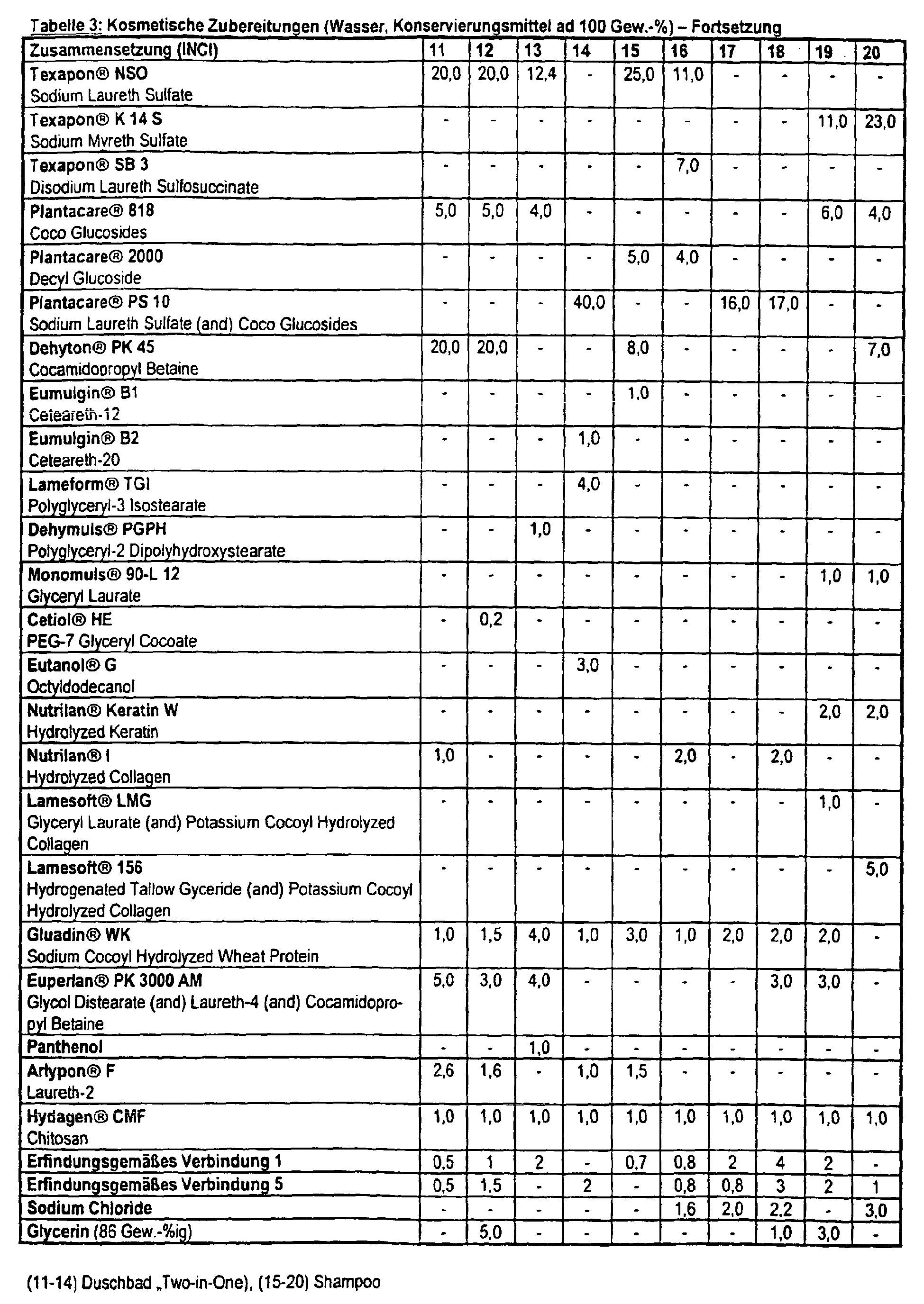

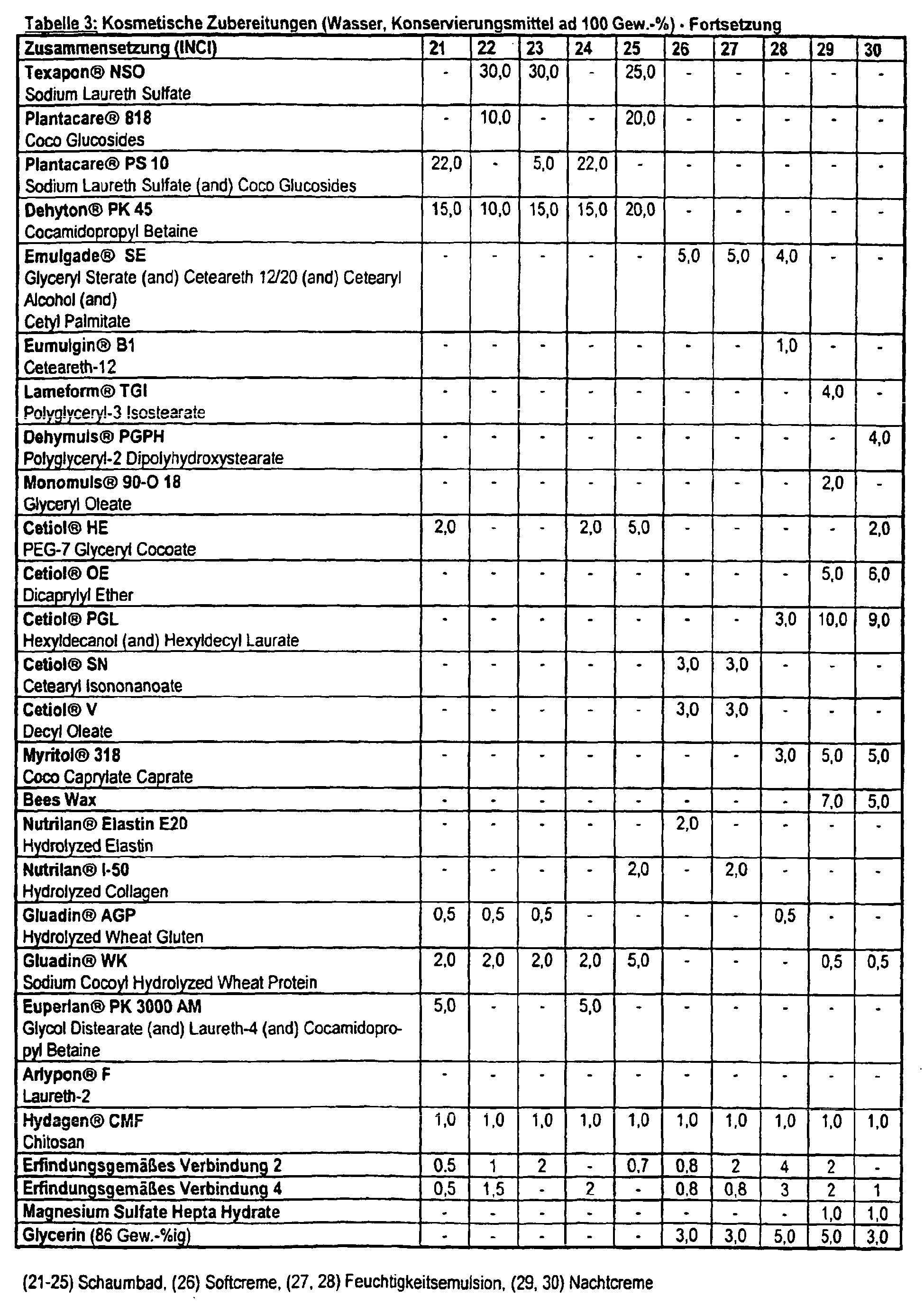

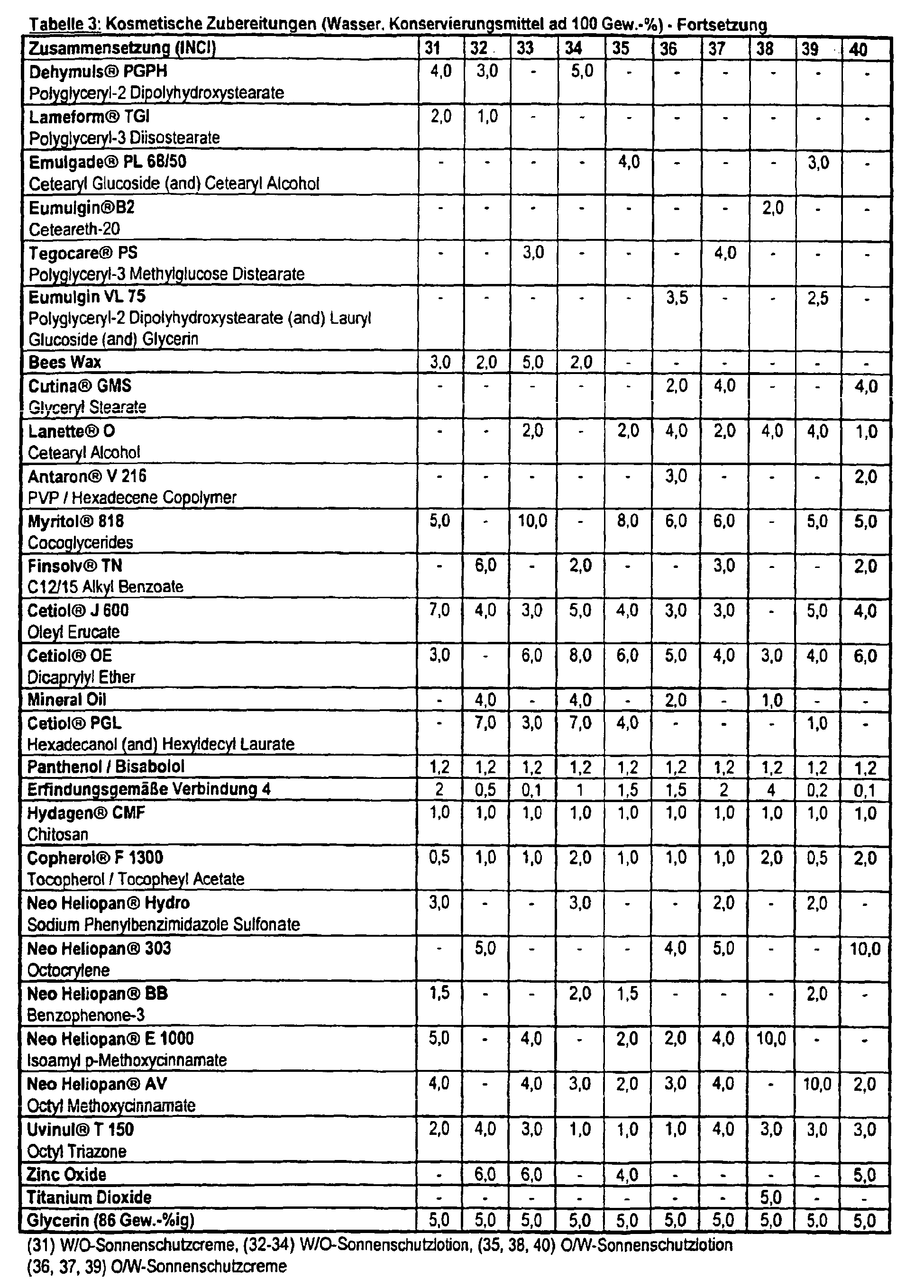

## Patentansprüche

1. Verwendung von nanoskaligen Esterquats, Tetraalkylammoniumverbindungen und/oder kationischen Polymeren mit Teitchendurchmessem im Bereich von 10 bis 300 nm zur Herstellung von kosmetischen und/oder pharmazeutischen Zubereitungen.

2. Verwendung von nanoskaligen Esterquats. Tetraalkylammoniumverbindungen und/oder kationischen Polymeren mit Teilchendurchmessem im Bereich von 10 bis 300 nm zur Herstellung von Wäscheweichspüler.

3. Verwendung nach mindestens einem der Ansprüche 1 bis 2, **dadurch gekennzeichnet, daß** man Esterquats der Formel (I) einsetzt, 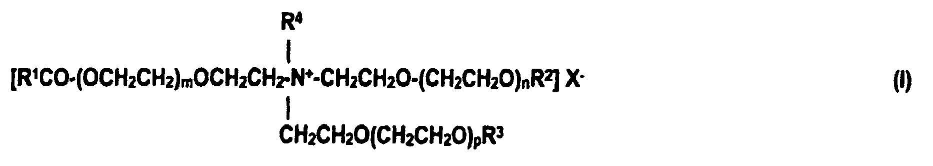 in der R¹CO für einen Acylrest mit 6 bis 22 Kohlenstoffatomen, R² und R³ unabhängig voneinander für Wasserstoff oder R¹CO, R⁴ für einen Alkylrest mit 1 bis 4 Kohlenstoffatomen oder eine (CH₂CH₂O)_{q}H-Gruppe, m, n und p in Summe für 0 oder Zahlen von 1 bis 12, q für Zahlen von 1 bis 12 und X für Halogenid, Alkylsulfat oder Alkylphosphat steht.

4. Verwendung nach mindestens einem der Ansprüche 1 bis 2, **dadurch gekennzeichnet, daß** man Esterquats der Formel (II) einsetzt, 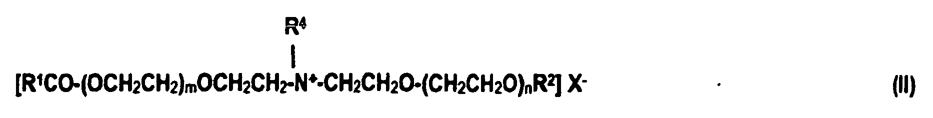 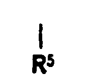 in der R¹CO für einen Acylrest mit 6 bis 22 Kohlenstoffatomen, R² für Wasserstoff oder R¹CO, R⁴ und R⁵ unabhängig voneinander für Alkylreste mit 1 bis 4 Kohlenstoffatomen, m und n in Summe für 0 oder Zahlen von 1 bis 12 und X für Halogenid, Alkylsulfat oder Alkylphosphat steht.

5. Verwendung nach mindestens einem der Ansprüche 1 bis 2, **dadurch gekennzeichnet, daß** man Esterquats der Formel (III) einsetzt, 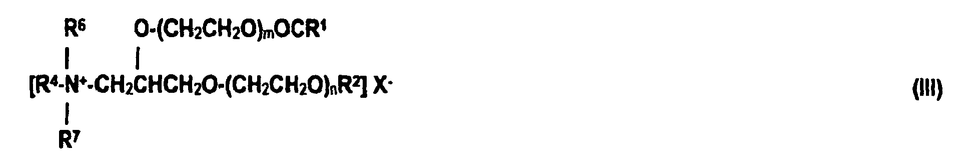 in der R¹CO für einen Acylrest mit 6 bis 22 Kohlenstoffatomen, R² für Wasserstoff oder R¹CO, R⁴, R⁶ und R⁷ unabhängig voneinander für Alkylreste mit 1 bis 4 Kohlenstoffatomen, m und n in Summe für 0 oder Zahlen von 1 bis 12 und X für Halogenid, Alkylsulfat oder Alkylphosphat steht.

6. Verwendung nach mindestens einem der Ansprüche 1 bis 2, **dadurch gekennzeichnet, daß** man Esterquats der Formel (IV) einsetzt, 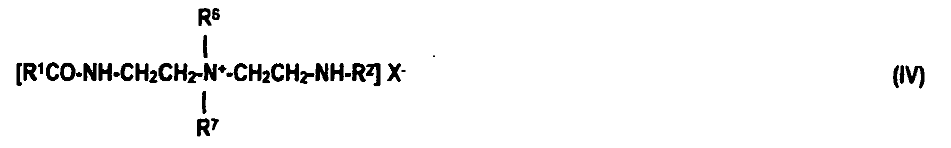 in der R¹CO für einen Acylrest mit 6 bis 22 Kohlenstoffatomen, R² für Wasserstoff oder R¹CO, R⁶ und R⁷ unabhängig voneinander für Alkylreste mit 1 bis 4 Kohlenstoffatomen und X für Halogenid, Alkylsulfat oder Alkylphosphat steht.

7. Verwendung nach mindestens einem der Ansprüche 1 bis 6, **dadurch gekennzeichnet, daß** man Esterquats der Formel (V) einsetzt, 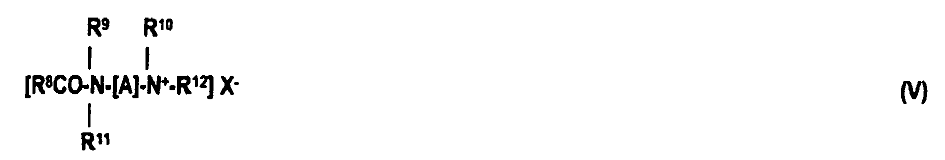 in der R⁸CO für einen gesättigten und/oder ungesättigten ethoxylierten Hydroxyacylrest mit 16 bis 22, vorzugsweise 18 Kohlenstoffatomen sowie 1 bis 50 Oxyethyleneinheiten, A für einen linearen oder verzweigten Alkylenrest mit 1 bis 6 Kohlenstoffatomen, R⁹, R¹⁰ und R¹¹ unabhängig voneinander für Wasserstoff oder eine Alkylgruppe mit 1 bis 4 Kohlenstoffatomen, R¹² für einen Alkylrest mit 1 bis 4 Kohlenstoffatomen oder einen Benzylrest und X für Halogen, Alkylsulfat oder Alkylphosphat steht.

8. Verwendung nach mindestens einem der Ansprüche 1 bis 2, **dadurch gekennzeichnet, daß** man Tetraalkylammoniumverbindungen der Formel (VI) einsetzt, 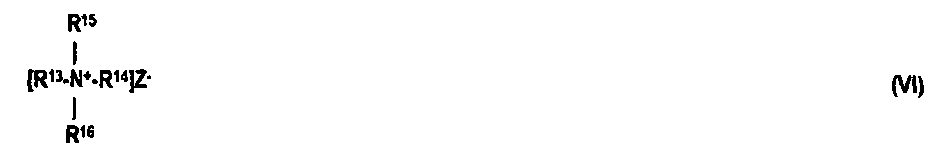 in der R¹³ für einen linearen oder verzweigten, gegebenenfalls hydroxysubstituierten Alkylrest mit 6 bis 22 Kohlenstoffatomen oder einen Benzylrest, R¹⁴ und R¹⁵ unabhängig voneinander für einen gegebenenfalls hydroxysubstituierten Alkylrest mit 1 bis 22 Kohlenstoffatomen, R¹⁶ für einen gegebenenfalls hydroxysubstituierten Alkylrest mit 1 bis 4 Kohlenstoffatomen und Z für Halogenid, Alkylsulfat oder Alkylphosphat steht.

9. Verwendung nach mindestens einem der Ansprüche 1 bis 2, **dadurch gekennzeichnet, daß** man kationische Polymere einsetzt, die ausgewählt sind aus der Gruppe, die gebildet wird von kationische Cellulosederivate, kationische Stärke, Copolymere von Diallylammoniumsalzen und Acrylamiden, quaternierte Vinylpyrrolidon/Vinylimidazol-Polymere, Kondensationsprodukte von Polyglycolen und Aminen, quatemierte Kollagenpolypepide, quatemierte Weizenpolypeptide, Polyethylenimin, kationische Siliconpolymere, Copolymere der Adipinsäure und Dimethylaminohydroxypropyldiethylentriamin, Copolymere der Acrylsäure mit Dimethyldiallylammoniumchlorid, Polyaminopolyamide sowie deren vemetzte wasserlöslichen Polymere, kationische Chitinderivate, gegebenenfalls mikrokristallin verteilt, Kondensationsprodukte aus Dihalogenalkylen, kationischer Guar-Gum, quatemierte Ammoniumsalz-Polymere.

10. Verwendung nach mindestens einem der Ansprüche 1 bis 9, **dadurch gekennzeichnet, daß** man kationischen Verbindungen einsetzt, die man erhält, indem man
(a) die Ausgangsstoffe unter überkritischen oder nahekritischen Bedingungen in einem geeigneten Lösungsmittel löst,
(b) die fluide Mischung über eine Düse in ein Vakuum, ein Gas oder eine Flüssigkeit entspannt, und
(c) das Lösemittel dabei gleichzeitig verdampft.

11. Verwendung nach mindestens einem der Ansprüche 1 bis 10, **dadurch gekennzeichnet, daß** man Nanopartikel einsetzt, welche von einem Schutzkolloid ummantelt vorliegen.

12. Verwendung nach Anspruch 11, **dadurch gekennzeichnet, daß** man als Schutzkolloid Polyvinylalkohol oder Polyethylenglycol einsetzt.

13. Verwendung nach mindestens einem der Ansprüche 1 bis 12, **dadurch gekennzeichnet, daß** man die kationischen Verbindungen in Mengen von 0,1 bis 10 Gew.% - bezogen auf die Zubereitungen - einsetzt.

## Claims

1. The use of nanoscale esterquats, tetraalkyl ammonium compounds and/or cationic polymers with particle diameters of 10 to 300 nm for the production of cosmetic and/or pharmaceutical preparations.

2. The use of nanoscale esterquats, tetraalkyl ammonium compounds and/or cationic polymers with particle diameters of 10 to 300 nm for the production of fabric softeners.

3. The use claimed in at least one of claims 1 to 2, **characterized in that** esterquats corresponding to formula (I): 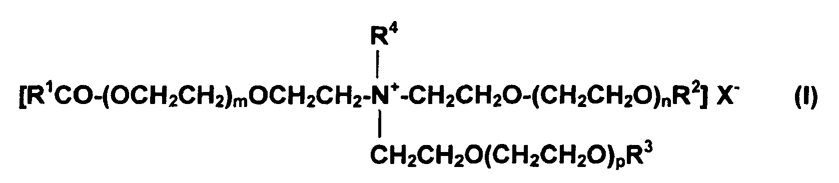 in which R¹CO is an acyl group containing 6 to 22 carbon atoms, R² and R³ independently of one another represent hydrogen or have the same meaning as R¹CO, R⁴ is an alkyl group containing 1 to 4 carbon atoms or a (CH₂CH₂O)_{q}H group, m, n and p together stand for 0 or numbers of 1 to 12, q is a number of 1 to 12 and X is halide, alkyl sulfate or alkyl phosphate,
are used.

4. The use claimed in at least one of claims 1 to 2, **characterized in that** esterquats corresponding to formula (II): 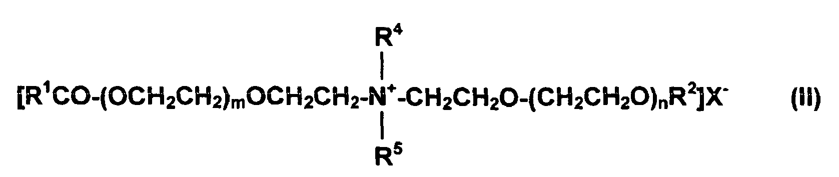 in which R¹CO is an acyl group containing 6 to 22 carbon atoms, R² is hydrogen or has the same meaning as R¹CO, R⁴ and R⁵ independently of one another are alkyl groups containing 1 to 4 carbon atoms, m and n together stand for 0 or numbers of 1 to 12 and X stands for halide, alkyl sulfate or alkyl phosphate,
are used.

5. The use claimed in at least one of claims 1 to 2, **characterized in that** esterquats corresponding to formula (III): 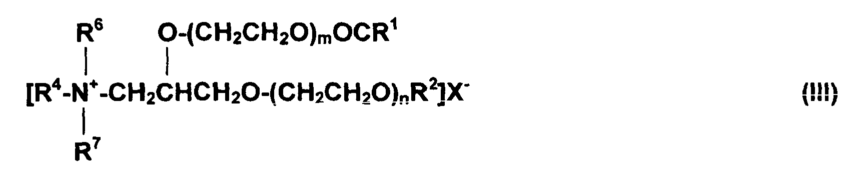 in which R¹CO is an acyl group containing 6 to 22 carbon atoms, R² is hydrogen or has the same meaning as R¹CO, R⁴, R⁶ and R⁷ independently of one another are alkyl groups containing 1 to 4 carbon atoms, m and n together stand for 0 or numbers of 1 to 12 and X stands for halide, alkyl sulfate or alkyl phosphate,
are used.

6. The use claimed in at least one of claims 1 to 2, **characterized in that** esterquats corresponding to formula (IV): 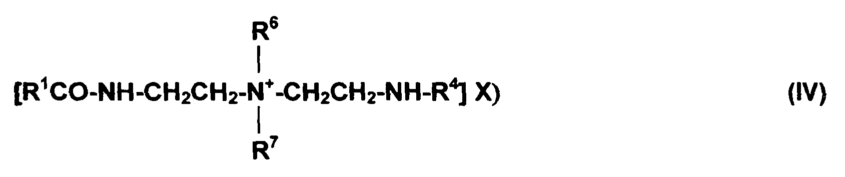 in which R¹CO is an acyl group containing 6 to 22 carbon atoms, R² is hydrogen or has the same meaning as R¹CO, R⁶ and R⁷ independently of one another are alkyl groups containing 1 to 4 carbon atoms and X is halide, alkyl sulfate or alkyl phosphate,
are used.

7. The use claimed in at least one of claims 1 to 6, **characterized in that** esterquats corresponding to correspond to formula (V): 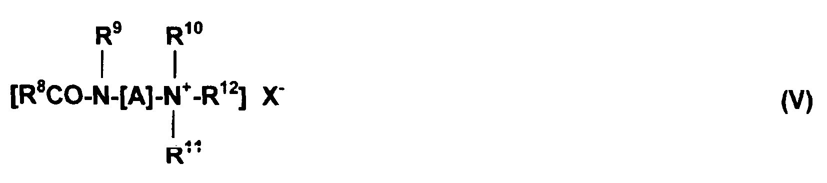 in which R⁸CO is a saturated and/or unsaturated ethoxylated hydroxyacyl group containing 16 to 22 and preferably 18 carbon atoms and 1 to 50 oxyethylene units, A is a linear or branched alkylene group containing 1 to 6 carbon atoms, R⁹, R¹⁰ and R¹¹ independently of one another represent hydrogen or a C₁₋₄ alkyl group, R¹² is a C₁₋₄ alkyl group or a benzyl group and X is halogen, alkyl sulfate or alkyl phosphate,
are used.

8. The use claimed in at least one of claims 1 to 2, **characterized in that** tetraalkyl ammonium compounds corresponding to formula (VI): 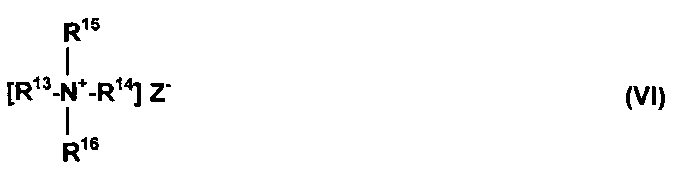 in which R¹³ is a linear or branched, optionally hydroxysubstituted alkyl group containing 6 to 22 carbon atoms or a benzyl radical, R¹⁴ and R¹⁵ independently of one another represent an optionally hydroxysubstituted alkyl group containing 1 to 22 carbon atoms, R¹⁶ is an optionally hydroxysubstituted alkyl group containing 1 to 4 carbon atoms and Z is halide, alkyl sulfate or alkyl phosphate,
are used.

9. The use claimed in at least one of claims 1 to 2, **characterized in that** cationic polymers selected from the group consisting of cationic cellulose derivatives, cationic starch, copolymers of diallyl ammonium salts and acrylamides, quaternized vinyl pyrrolidone/vinyl imidazole polymers, condensation products of polyglycols and amines, quaternized collagen polypeptides, quaternized wheat polypeptides, polyethyleneimine, cationic silicone polymers, copolymers of adipic acid and dimethyl aminohydroxypropyl diethylenetriamine, copolymers of acrylic acid with dimethyl diallyl ammonium chloride, polyaminopolyamides and crosslinked water-soluble polymers thereof, cationic chitin derivatives, optionally in microcrystalline distribution, condensation products of dihaloalkyls, cationic guar gum, quaternized ammonium salt polymers are used.

10. The use claimed in at least one of claims 1 to 9, **characterized in that** cationic compounds obtained by
(a) dissolving the starting materials in a suitable solvent under supercritical or near-critical conditions,
(b) expanding the fluid mixture through a nozzle into a vacuum, a gas or a liquid and
(c) simultaneously evaporating the solvent
are used.

11. The use claimed in at least one of claims 1 to 10, **characterized in that** nanoparticles coated with a protective colloid are used.

12. The use claimed in claim 11, **characterized in that** polyvinyl alcohol or polyethylene glycol is used as the protective colloid.

13. The use claimed in at least one of claims 1 to 12, **characterized in that** the cationic compounds are used in quantities of 0.1 to 10% by weight, based on the preparations.

## Revendications

1. Utilisation d'esters d'ammonium quaternaire de composés de tétraalkylammonium et/ou de polymères cationiques à l'échelle nanométrique, ayant des diamètres de particules compris entre 10 à 300 nm pour la préparation de préparations cosmétiques et/ou pharmaceutiques.

2. Utilisation d'esters d'ammonium quaternaire, de composés de tétraalkylammonium et/ou de polymères cationiques à l'échelle nanométrique ayant des diamètres de particules compris entre 10 à 300 nm pour la préparation d'assouplissants.

3. Utilisation selon au moins l'une des revendications 1 et 2,
**caractérisée en ce qu'**
on utilise des esters d'ammonium quaternaire de formule (I) 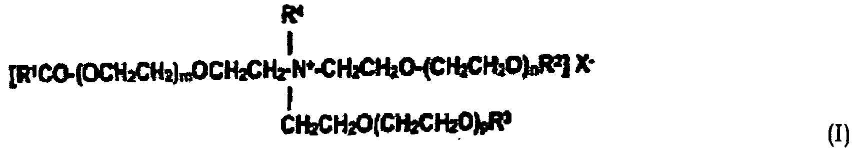 dans laquelle R¹CO représente un radical acyle comportant de 6 à 22 atomes de carbone ; R² et R³ représentent, indépendamment l'un de l'autre, l'hydrogène ou R¹CO ; R⁴ représente un radical alkyle comportant de 1 à 4 atomes de carbone ou un groupe (CH₂CH₂O)qH m, n et p ont pour somme soit 0 soit des nombres allant de 1 à 12 ; q représente des nombres de 1 à 12, et X représente un halogénure, un sulfate d'alkyle ou un phosphate d'alkyle.

4. Utilisation selon au moins l'une des revendications 1 et 2,
**caractérisée en ce qu'**
on utilise des esters d'ammonium quaternaire de formule (II) 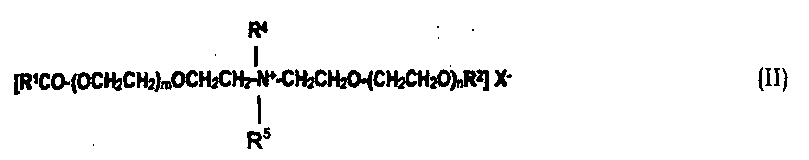 dans laquelle R¹CO représente un radical acyle comportant de 6 à 22 atomes de carbone ; R² représente l'hydrogène ou R¹CO; R⁴ et R⁵ représentent, indépendamment l'un de l'autre, des radicaux alkyle comportant de 1 à 4 atomes de carbone ; m et n ont pour somme soit 0 soit des nombres de 1 à 12 ; et X représente un halogénure, un sulfate d'alkyle ou un phosphate d'alkyle.

5. Utilisation selon au moins l'une des revendications 1 et 2,
**caractérisée en ce que**
on utilise des esters d'ammonium quaternaire de formule (III) 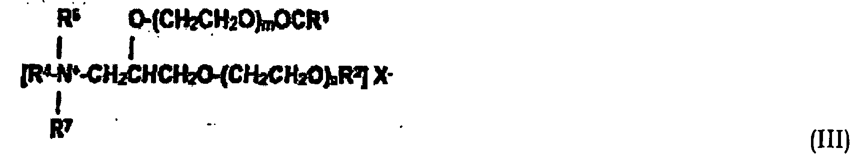 dans laquelle R¹CO représente un radical acyle comportant de 6 à 22 atomes de carbone ; R² représente l'hydrogène ou R¹CO ; R⁴, R⁶ et R⁷ représentent, indépendamment les uns des autres, des radicaux alkyle comportant de 1 à 4 atomes de carbone ; m et n ont pour somme soit 0 soit des nombres allant de 1 à 12 ; et X représente un halogénure, un sulfate d'alkyle ou un phosphate d'alkyle.

6. Utilisation selon au moins l'une des revendications 1 et 2,
**caractérisée en ce qu'**
on utilise des esters d'ammonium quaternaire de formule (IV) 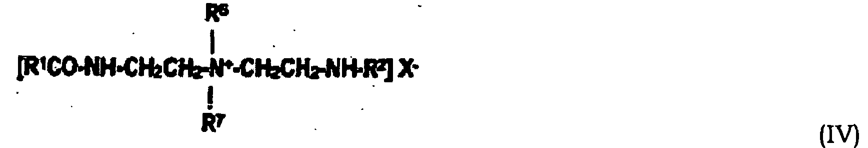 dans laquelle R¹CO représente un radical acyle comportant de 6 à 22 atomes de carbone ; R² représente l'hydrogène ou R¹CO; R⁶ et R⁷ représentent, indépendamment l'un de l'autre, des radicaux alkyle comportant de 1 à 4 atomes de carbone ; et X représente un halogénure, un sulfate d'alkyle ou un phosphate d'alkyle.

7. Utilisation selon au moins l'une des revendications 1 et 2,
**caractérisée en ce qu'**
on utilise des esters d'ammonium quaternaire de formule (V) 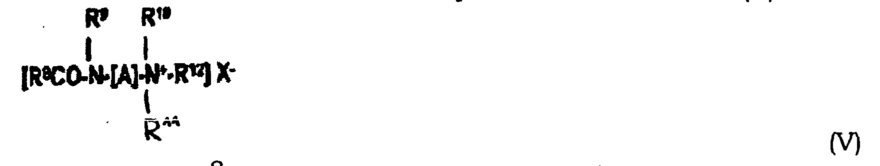 dans laquelle R⁸CO représente un radical hydroxyacyle éthoxylé saturé et/ou insaturé, comportant de 16 à 22, de préférence 18 atomes de carbone et de 1 à 50 unités d'oxyde éthylène ; A représente un radical alkylène, linéaire ou ramifié, comportant de 1 à 6 atomes de carbone; R⁹, R¹⁰ et R¹¹ représentent, indépendamment les uns des autres, l'hydrogène ou un groupe alkyle comportant de 1 à 4 atomes de carbone ; R¹² représente un radical alkyle comportant de 1 à 4 atomes de carbone ou un radical benzyle ; et X représente un halogénure, un sulfate d'alkyle ou un phosphate d'alkyle.

8. Utilisation selon au moins l'une des revendications 1 et 2,
**caractérisée en ce qu'**
on utilise des composés de tétraalkylammonium de formule (VI) 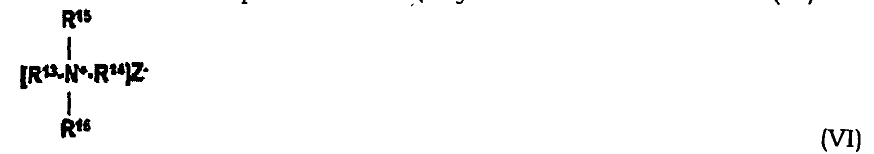 dans laquelle R¹³ représente un radical alkyle linéaire ou ramifié, le cas échéant hydroxysubstitué, comportant de 6 à 22 atomes de carbone, ou un radical benzyle ; R¹⁴ et R¹⁵ représentent, indépendamment l'un de l'autre, un radical alkyle le cas échéant hydroxysubstitué, comportant de 1 à 22 atomes de carbone ; R¹⁶ représente un radical alkyle le cas échéant hydroxysubstitué comportant de 1 à 4 atomes de carbone ; et Z représente un halogénure, un sulfate d'alkyle ou un phosphate d'alkyle.

9. Utilisation selon au moins l'une des revendications 1 et 2,
**caractérisée en ce qu'**
on utilise des polymères cationiques choisis dans le groupe formé par les substances suivantes : dérivés cationiques de la cellulose, amidon cationique, copolymères de sels de diallylammonium et d'acrylamides, polymères de vinylpyrrolidone/vinylimidazol, quaternisés produits de condensation de polyglycols et d'amines, polypeptides de collagène quaternisés, polypeptides de froment quaternisés, polyéthylèneimine, polymères de silicone cationiques, copolymères de l'acide adipique et de la diméthylaminohydroxypropyldiéthylènetriamine, copolymères de l'acide acrylique et du chlorure de diméthyldiallylammonium, polyaminopolyamides et leurs polymères réticulés solubles dans l'eau, dérivés cationiques de la chitine répartis le cas échéant sous forme microcristalline, produits de condensation de dihalogénures d'alkylène, gomme guar cationique, polymères de sels d'ammonium quaternisés

10. Utilisation selon au moins l'une des revendications 1 à 9,
**caractérisée en ce qu'**
on utilise des composés cationiques que l'on obtient
(a) en dissolvant les produits de départ dans un solvant approprié dans des conditions surcritiques ou sensiblement critiques,
(b) en détendant le mélange fluide, dans un vide, un gaz ou un liquide par l'intermédiaire d'une buse, et
(c) en faisant simultanément évaporer le solvant.

11. Utilisation selon au moins l'une des revendications 1 à 10,
**caractérisée en ce qu'**
on utilise des nanoparticules enrobées d'un colloïde de protection.

12. Utilisation selon la revendication 11,
**caractérisée en ce qu'**
on utilise de l'alcool polyvinylique ou du polyéthylèneglycol comme colloïde de protection.

13. Utilisation selon au moins l'une des revendications 1 à 12,
**caractérisée en ce qu'**
on utilise les composés cationiques en quantités de 0,1 à 10 % en poids par rapport aux préparations.
